(19) Europäisches Patentamt / European Patent Office / Office européen des brevets

(11) **EP 4 311 865 A1**

(12) # EUROPEAN PATENT APPLICATION

(43) Date of publication:
**31.01.2024   Bulletin 2024/05**

(21) Application number: **22187836.6**

(22) Date of filing: **29.07.2022**

(51) International Patent Classification (IPC):
**C23C 16/40** (2006.01)    **C23C 16/515** (2006.01)
**C23C 14/04** (2006.01)

(52) Cooperative Patent Classification (CPC):
**C23C 16/401; C23C 14/046; C23C 16/515**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **SCHOTT Pharma AG & Co. KGaA**
**55122 Mainz (DE)**

(72) Inventors:
• **WOYWOD, Tanja**
**55126 Mainz (DE)**

• **GHECZY, Inga**
**65719 Hofheim am Taunus (DE)**
• **DJORDJEVIC-REIß, Jovana**
**55128 Mainz (DE)**
• **SCHNEIDER, Thorsten**
**55299 Nackenheim (DE)**

(74) Representative: **Fuchs Patentanwälte Partnerschaft mbB**
**Tower 185**
**Friedrich-Ebert-Anlage 35-37**
**60327 Frankfurt am Main (DE)**

(54) ## COATED ELEMENT AND METHOD FOR COATING AN ELEMENT

(57)    This disclosure relates to a coated element having a surface, wherein at least a part of the surface of the element is coated by a coating, a system or kit, comprising the coated element, a closed system comprising the coated element, and a method for coating an element.

The coated element is manufactured coated by plasma impulse CVD (PICVD) by a tri-layer coating system of a smoothing layer, barrier layer and protecting layer. The coated element provides the system or kit as a pharmaceutical package, like a vial or syringe with plunger.

EP 4 311 865 A1

| GL |
| PL |
| BL |
| SL |
| CE |

Figure 1A

**Description**

This disclosure relates to a coated element having a surface, wherein at least a part of the surface of the element is coated by a coating, a system or kit, comprising the coated element, a closed system comprising the coated element, and a method for coating an element.

Background

Pharmaceutical compositions for injection, for example vaccines, are commonly administered by means of a syringe to a patient. Therefore, the pharmaceutical composition might be accommodated in a vial and is only drawn in the syringe a few minutes before injection, or alternatively the pharmaceutical composition is directly filled in a syringe after production and is accommodated in the syringe until administration. In case the pharmaceutical composition is directly filled in the syringe after production, the pharmaceutical composition is in direct contact with the inner surface of the syringe over a long time. Thereby, the pharmaceutical composition may interact with the surface of the syringe or air might diffuse through the wall of the syringe. As a consequence, the efficacy of the pharmaceutical composition might decrease or is contaminated with extractables and leachables. To decrease the interaction and suppress the diffusion of air, the surface of the syringe might be coated with a coating. However, these coatings are commonly very sensitive to high or low pH values and may itself contaminate the pharmaceutical composition if the coating peels of or dissolves. The inventors recognized these issues and thus, it is an object of the present application to provide a coated element having sufficient barrier properties and improved resistance and a method for the production said coated element. In detail, it is an object of the present invention to provide a coated element exhibiting one or more of:

- improved barrier properties with regard to air, especially oxygen;

- improved pH resistance;

- reduced amount of extractables;

- reduced amount of leachables;

- reduced leachable species;

- reduced delamination;

- improved adhesion of the coating or the coating and the decor; and/or

- improved gliding properties of the coating.

In addition, it is an objection of the present invention to provide an improved method for the production of a coated element. In detail, it is an object of the present invention to provide a method for the production of a coated substrate, preferably according to any embodiment described herein, exhibiting one or more of:

- increased applying speed;

- reduces costs;

- increased production reliability; and/or

- reduced production fluctuations.

Summary of the invention

The claimed subject-matter solves one or more of the above objects.
In one aspect, this disclosure relates to a coated element having a surface, wherein at least a part of the surface of the element is coated by a coating, wherein the coating comprises, in this order:

- a smoothing layer (SL);

- a barrier layer (BL); and

- a protection layer (PL),

wherein the smoothing layer satisfies the condition $a1_{SL} \leq [Si_2O^+]_{SL}/[SiC_3H_9^+]_{SL} \leq a2_{SL}$, wherein $a1_{SL}$ is 1.0, wherein $a2_{SL}$ is $1*10^5$, wherein $[SiC_3H_9^+]_{SL}$ are the counts of $[SiC_3H_9^+]$ ions in the smoothing layer, measured by a TOF-SIMS (Time-of-Flight Secondary-Ion-Mass-Spectrometry), wherein $[Si_2O^+]_{SL}$ are the counts of $[Si_2O^+]$ ions in the smoothing layer, measured by a TOF-SIMS, and/or

wherein the barrier layer satisfies the condition $a1_{BL} \leq [Si_2O^+]_{BL}/[SiC_3H_9^+]_{BL} \leq a2_{BL}$, wherein $a1_{BL}$ is 1, wherein $a2_{BL}$ is $1*10^5$, wherein $[SiC_3H_9^+]_{BL}$ are the counts of $[SiC_3H_9^+]$ ions in the barrier layer, measured by a TOF-SIMS, and wherein $[Si_2O^+]_{BL}$ are the counts of $[Si_2O^+]$ ions in the barrier layer, measured by a TOF-SIMS, and/or

wherein the protection layer satisfies the condition $a1_{PL} \leq [Si_2O^+]_{PL}/[SiC_3H_9^+]_{PL} \leq a2_{PL}$, wherein $a1_{PL}$ is 1.0, wherein $a2_{PL}$ is $1*10^{10}$, wherein $[SiC_3H_9^+]_{PL}$ are the counts of $[SiC_3H_9^+]$ ions in the protection layer, measured by a TOF-SIMS; and wherein $[Si_2O^+]_{PL}$ are the counts of $[Si_2O^+]$ ions in the protection layer, measured by a TOF-SIMS,

wherein the coated element optionally has a gliding force of 0.1 to 10.0 N, and/or a breaking force of 1 N to 18 N.

In another aspect, this disclosure relates to a coated element having a surface, wherein at least a part of the surface of the element is coated by a coating, wherein the coating comprises, in this order:

- a smoothing layer (SL);

- a barrier layer (BL); and

- a protection layer (PL),

wherein the smoothing layer has a thickness of 1 nm or more, and/or

wherein the barrier layer has a thickness of 1 nm or more, and/or

wherein the protection layer has a gliding force of 0.1 to 10.0 N, and/or a breaking force of 1 N to 18 N, and/or

wherein the coated element has a gliding force of 0.1 to 10.0 N, and/or a breaking force of 1 N to 18 N.

It is advantageous if the coating comprises a smoothing layer (SL), a barrier layer (BL) and a protection layer (PL), because the interaction of a pharmaceutical composition and the surface of the element may then be significantly reduced. The smoothing layer (SL) provides for an optimal contact and interaction with the (coated) element. The barrier layer (BL) is characterised by the predominance of inorganic species which may be provided by high temperatures in combination with oxygen employed during the coating process, e.g. using a PICVD method which generates a plasma. The barrier layer (BL) strongly reduces the oxygen transmission rate within and across the coating. The protection layer (PL) may be chemically similar to the smoothing layer (SL) and protects the barrier layer (BL).

In another aspect, this disclosure relates to a coated element having a surface, wherein at least a part of the surface of the element is coated by a coating, wherein the coating comprises, in this order:

- a smoothing layer (SL);

- a barrier layer (BL); and

- a protection layer (PL),

wherein $b1_{PL} \leq [SiC_2H^+]_{PL}/[SiO^+]_{PL} \leq b2_{PL}$, wherein $b1_{PL}$ is 0.5, wherein $b2_{PL}$ is $1*10^5$, wherein $[SiC_2H^+]_{PL}$ are the counts of $[SiC_2H^+]$ ions in the protection layer, measured by a TOF-SIMS; and wherein $[SiO^+]_{PL}$ are the counts of $[SiO^+]$ ions in the protection layer, measured by a TOF-SIMS.

In another aspect, this disclosure relates to a coated element having a surface, wherein at least a part of the surface of the element is coated by a coating, wherein the coating comprises, in this order:

- a smoothing layer (SL);

- a barrier layer (BL); and

- a protection layer (PL),

wherein $b1_{BL} \leq [SiO^+]_{BL}/[SiC_2H^+]_{BL} \leq b2_{BL}$, wherein $b1_{BL}$ is 1, wherein $b2_{BL}$ is $1*10^5$, wherein $[SiC_2H^+]_{BL}$ are the counts of $[SiC_2H^+]$ ions in the barrier layer, measured by a TOF-SIMS, wherein $[SiO^+]_{BL}$ are the counts of $[SiO^+]$ ions in the barrier layer, measured by a TOF-SIMS.

In another aspect, this disclosure relates to a coated element having a surface, wherein at least a part of the surface of the element is coated by a coating, wherein the coating comprises, in this order:

- a smoothing layer (SL);

- a barrier layer (BL); and

- a protection layer (PL),

wherein $b1_{SL} \leq [SiC_2H^+]_{SL}/[SiO^+]_{SL} \leq b2_{SL}$, wherein $b1_{SL}$ is 1.0, wherein $b2_{SL}$ is $1*10^5$, wherein $[SiC_2H^+]_{SL}$ are the counts of $[SiC_2H^+]$ ions in the smoothing layer, measured by a TOF-SIMS, wherein $[SiO^+]_{SL}$ are the counts of $[SiO^+]$ ions in the smoothing layer, measured by a TOF-SIMS.

In another aspect, this disclosure relates to a coated element having a surface, wherein at least a part of the surface of the element is coated by a coating, wherein the coating comprises, in this order:

- a smoothing layer (SL);

- a barrier layer (BL); and

- a protection layer (PL),

wherein the thickness of the smoothing layer is 1 nm to 20000 nm, or 5 nm to 5000, or 100 nm to 2000nm, or 150 nm to 1500nm, or 200 nm to 1000nm, or 250 nm to 500nm.

In a further aspect, this disclosure relates to a system or kit, comprising the coated element according to this disclosure, wherein the element is a syringe or cartridge; a plunger, preferably a plunger rod; and optionally a closure, preferably a tip cap and/or a needle shield.

In a further aspect, this disclosure relates to a closed system comprising:

the coated element according to this disclosure, wherein the element is a syringe or cartridge;

a plunger, preferably a plunger rod; and

a closure, preferably a tip cap and/or a needle shield,

wherein the closed coated element passes the container closure integrity test according to DIN EN ISO 8871-5:2016; chapter 4.4 in combination with Annex D.

In a further aspect, this disclosure relates to a method for coating an element, preferably a coated element according to this disclosure, comprising the steps:

(1) providing an element comprising a surface;

(2) applying a smoothing layer;

(3) applying a barrier layer;

(4) applying a protection layer; and

(5) optionally applying a gliding layer,

wherein each of the steps (2) to (4) comprises surrounding at least part of the surface of

the element with a precursor, and

wherein each of the steps (2) to (4) comprises irradiating the precursor to generate a plasma using a PICVD method,

wherein the step (3) comprises establishing a flow rate of $O_2$ of 10 sccm or more during the irradiation of the precursor.

Advantageously, the method provides for coating an element, wherein the coated element comprises, preferably in this order, a smoothing layer, a barrier layer, a protection layer, and optionally a gliding layer. The method advantageously allows and provides for designing and adjusting the respective application steps such that the smoothing layer, the barrier layer, the protection layer, and optionally the gliding layer may be physically and/or chemically distinct. The method is versatile and may be adapted such that e.g. the barrier layer may predominantly display inorganic surface moieties and/or may have a very low oxygen transmission rate, and/or such that e.g. the protection layer protects the barrier layer, and/or such that e.g. the gliding layer (if present) may predominantly display organic surface moieties.

Description of the Figures

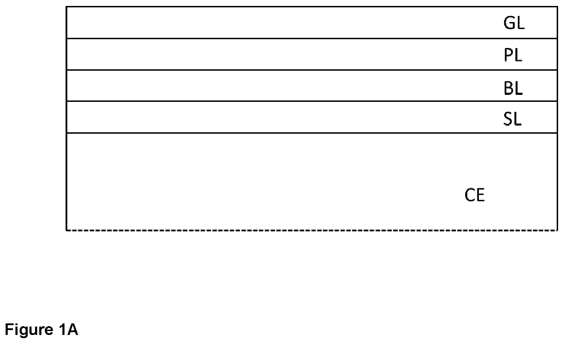

Figure 1A shows a schematic depiction of the layer structure of a coated element (CE) according to one embodiment of this disclosure, including a smoothing layer (SL), a barrier layer (BL), a protection layer (PL), and an optional gliding layer (GL), wherein the coated element may have a polymer substrate. The four different layers, i.e. SL, BL, PL and optionally GL, cover the coated element (CE) at least partially.

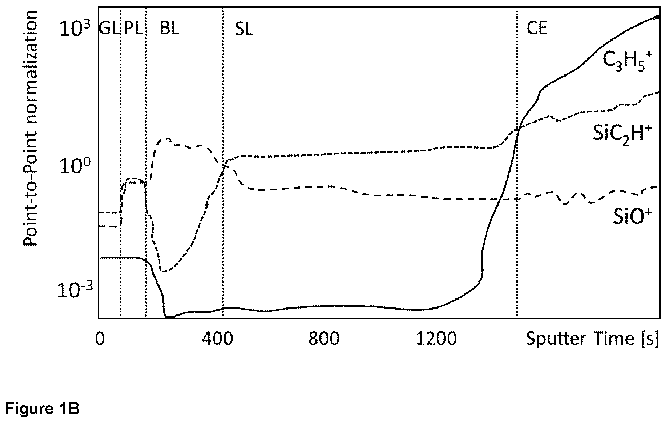

Figure 1B shows a schematic depiction of the TOF-SIMS data for the positively charged ion species $SiO^+$, $SiC_2H^+$ and $C_3H_5^+$ and indicates how the smoothing layer (SL), the barrier layer (BL), the protection layer (PL), and the gliding layer (GL) on the coated element (CE) are assigned based on this data. At the interface GL/PL, the point-to-point normalization values markedly change for the $SiO^+$ and the $SiC_2H^+$ species. At the interface PL/BL, the point-to-point normalization values markedly change for the $SiO^+$ and the $SiC_2H^+$ species. In the BL, the point-to-point normalization values become maximal for $SiO^+$, whereas the point-to-point normalization values become minimal for $SiC_2H^+$. In the coated element (CE), the species $C_3H_5^+$ becomes dominant, i.e. the point-to-point normalization values become maximal.

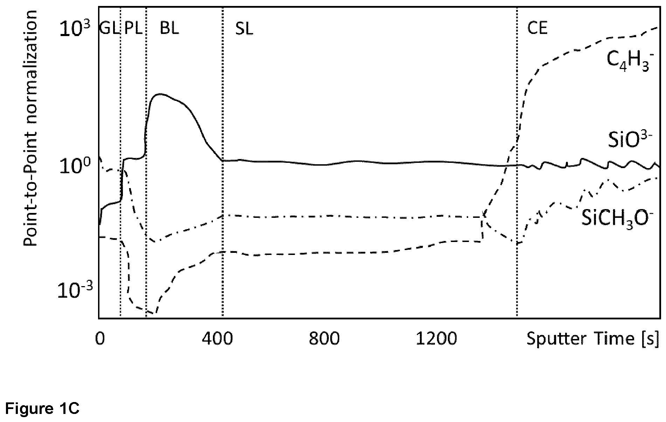

Figure 1C shows a schematic depiction of the TOF-SIMS data for the negatively charged ion species $SiCH_3O^-$, $SiO^{3-}$ and $C_4H_3^-$ and indicates how the smoothing layer (SL), the barrier layer (BL), the protection layer (PL), and the gliding layer (GL) on the coated element (CE) are assigned based on this data. At the interface GL/PL, the point-to-point normalization values markedly change for the $SiO^{3-}$ and the $SiCH_3O^-$ species. At the interface PL/BL, the point-to-point normalization values markedly change for the $SiO^{3-}$ species. In the BL, the point-to-point normalization values become maximal for $SiO^{3-}$, whereas the point-to-point normalization values become minimal for $SiCH_3O^-$. In the coated element (CE), the species $C_4H_3^-$ becomes dominant, i.e. the point-to-point normalization values become maximal.

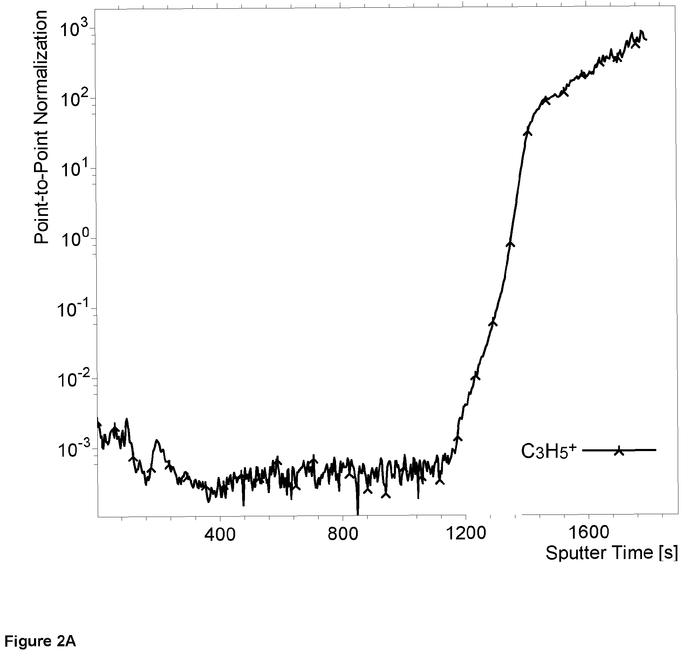

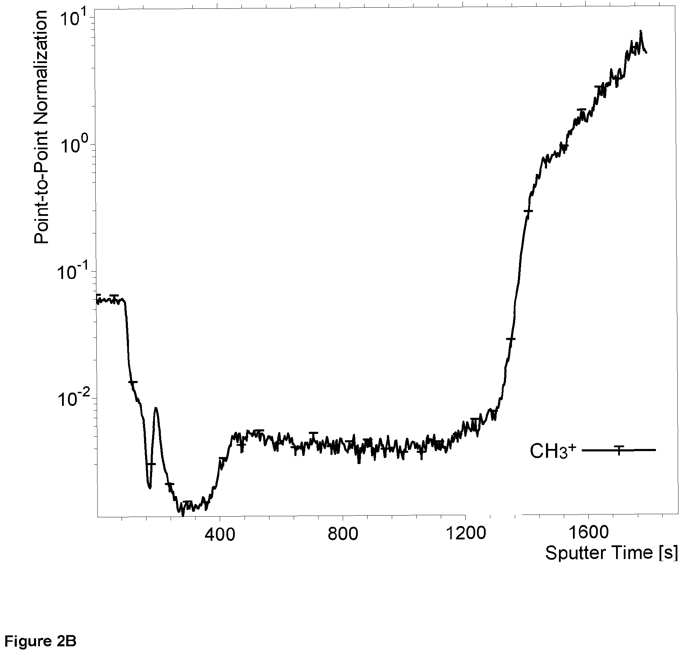

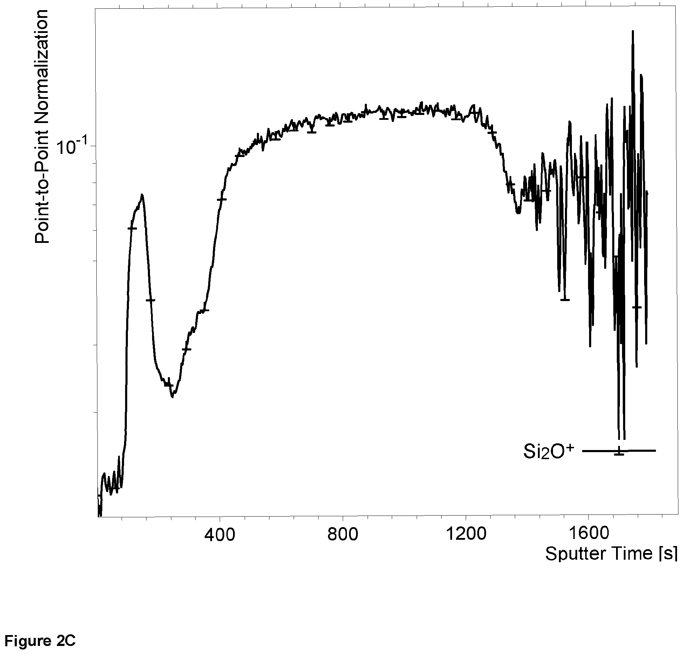

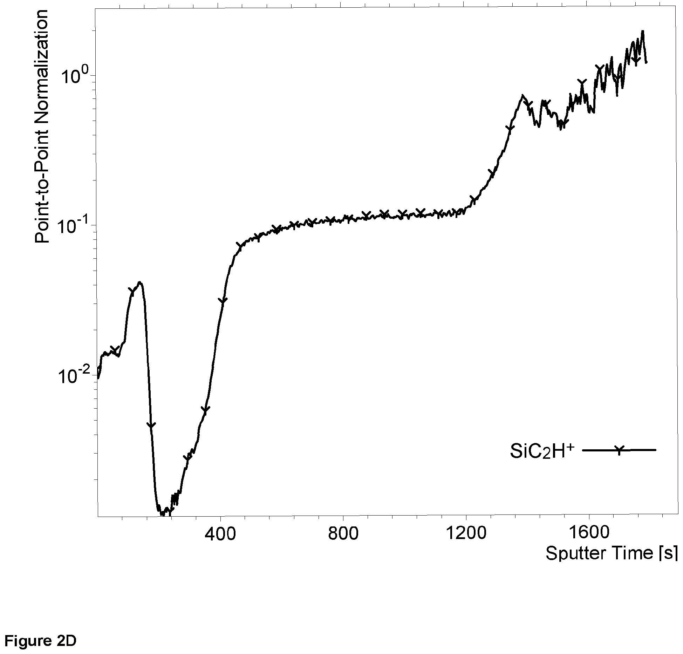

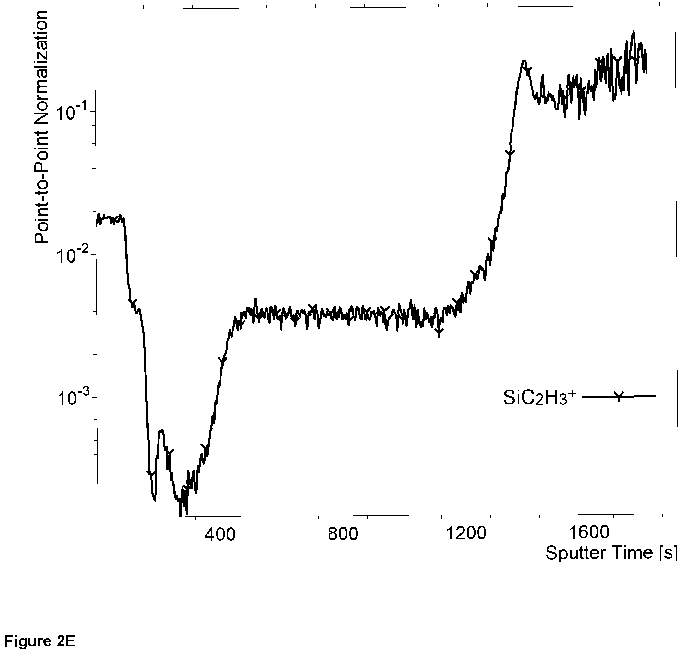

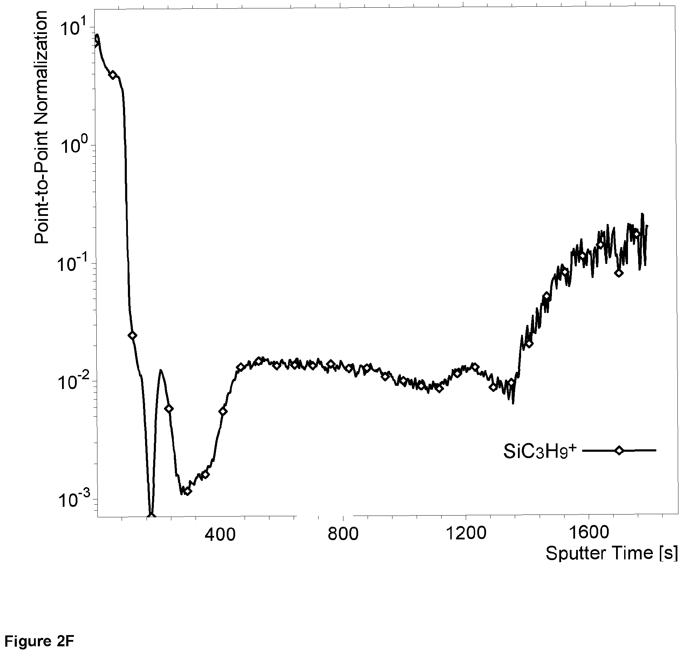

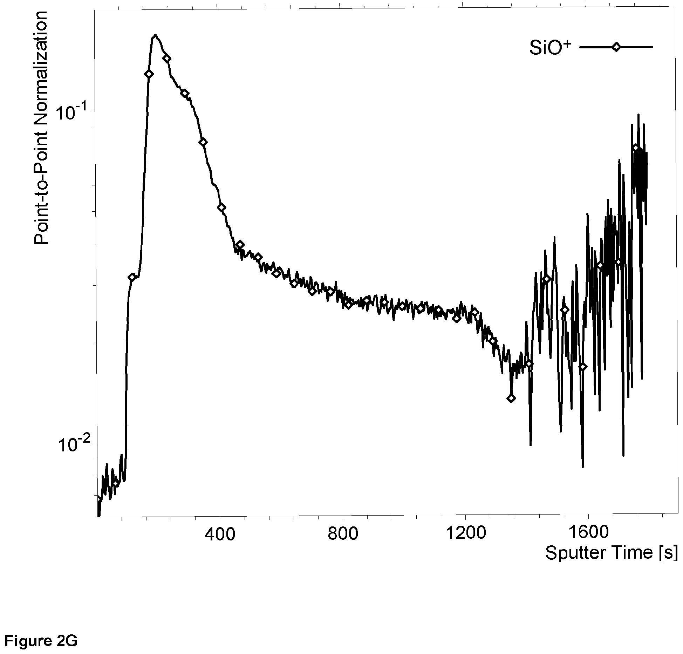

Figures 2A to 2G shows the results of example 1 of the herein described TOF-SIMS measurement (positive ions) displaying the species $C_3H_5^+$ (Figure 2A), $CH_3^+$ (Figure 2B), $Si_2O^+$ (Figure 2C), $SiC_2H^+$ (Figure 2D), $SiC_2H_3^+$ (Figure 2E), $SiC_3H_9^+$ (Figure 2F), and $SiO^+$ (Figure 2G).

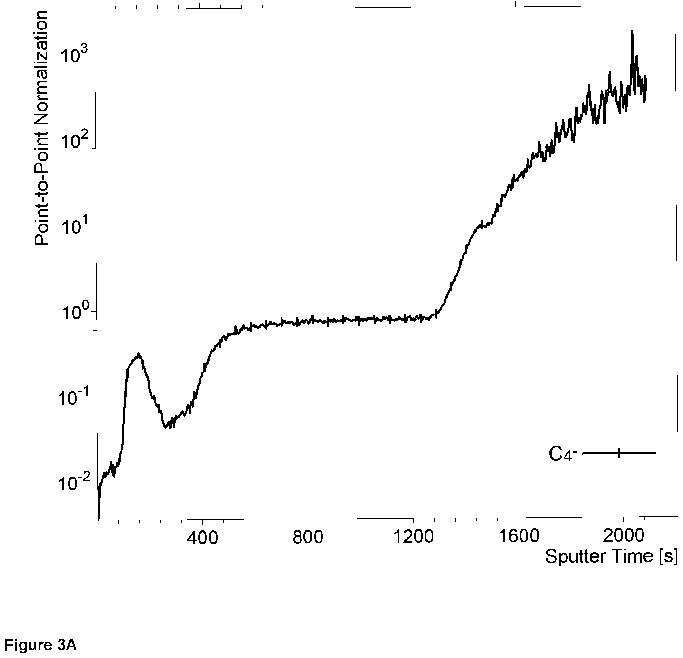

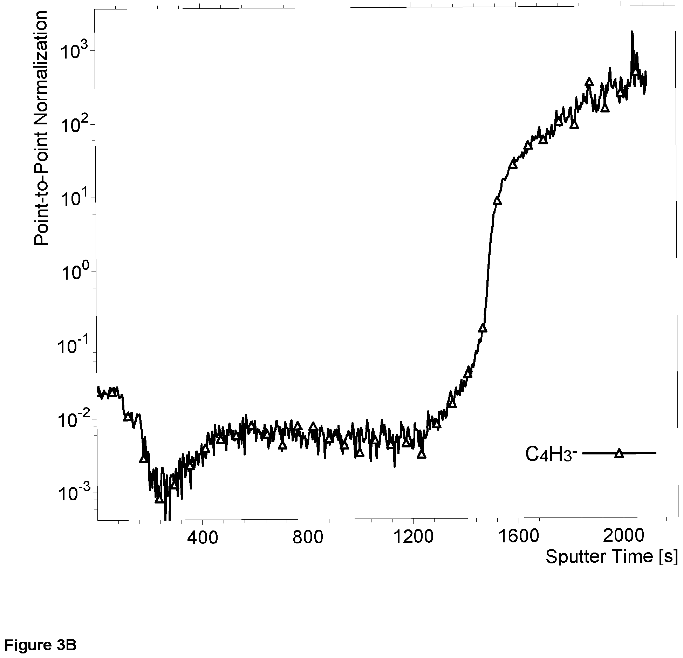

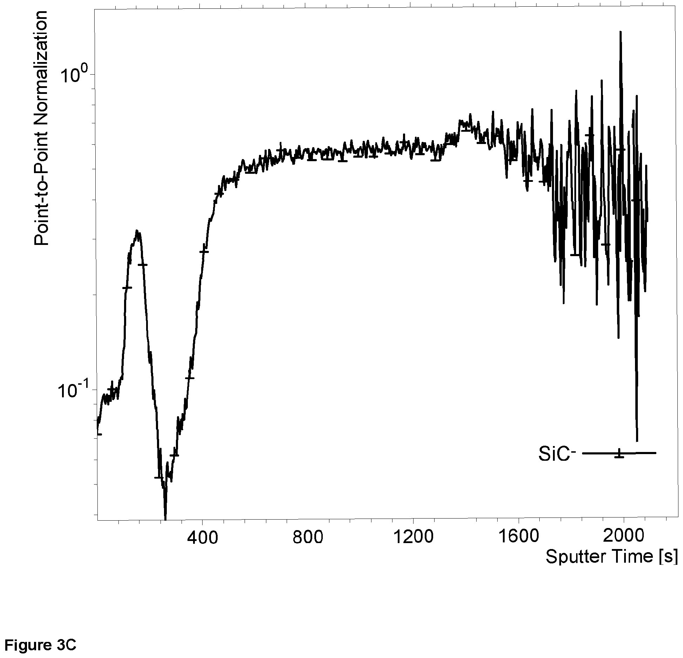

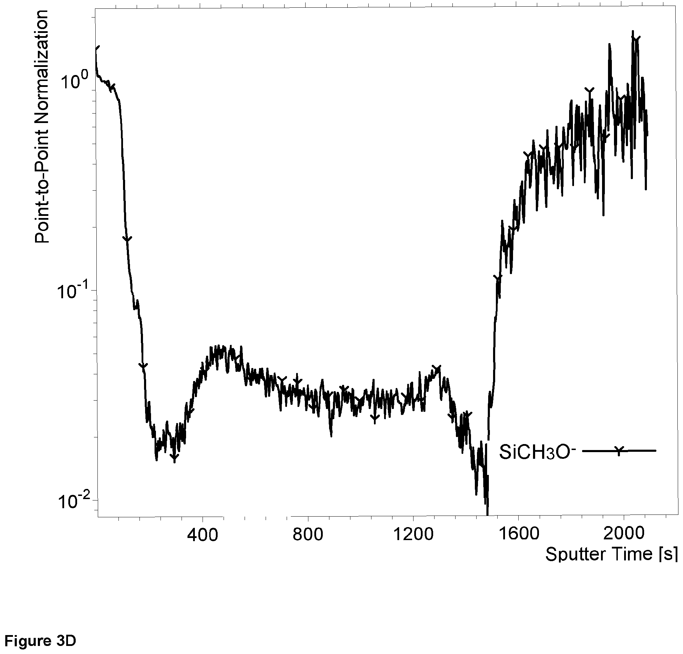

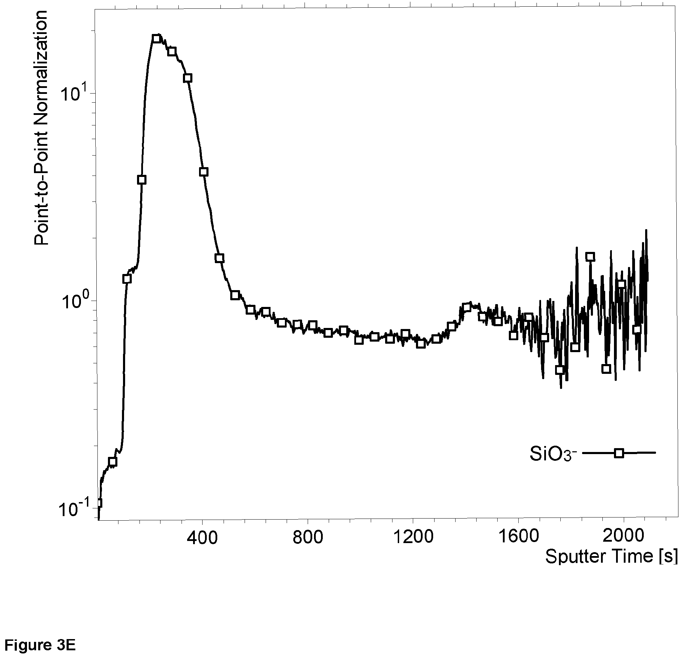

Figures 3A to 3E show the results of example 1 of the herein described TOF-SIMS measurement (negative ions) displaying the species $C_4^-$ (Figure 3A), $C_4H_3^-$ (Figure 3B), $SiC^-$ (Figure 3C), $SiCH_3O^-$ (Figure 3D) and $SiO_3^-$ (Figure 3E).

Detailed description of the invention

Claimed embodiments

In one aspect, this disclosure provides a coated element having a surface, wherein at least a part of the surface of the element is coated by a coating, wherein the coating comprises, in this order:

- a smoothing layer (SL);

- a barrier layer (BL); and

- a protection layer (PL),

wherein the smoothing layer satisfies the condition $a1_{SL} \leq [Si_2O^+]_{SL}/[SiC_3H_9^+]_{SL} \leq a2_{SL}$, wherein $a1_{SL}$ is 1.0, wherein $a2_{SL}$ is $1*10^5$, wherein $[SiC_3H_9^+]_{SL}$ are the counts of $[SiC_3H_9^+]$ ions in the smoothing layer, measured by a TOF-SIMS, wherein $[Si_2O^+]_{SL}$ are the counts of $[Si_2O^+]$ ions in the smoothing layer, measured by a TOF-SIMS, and/or

wherein the barrier layer satisfies the condition $a1_{BL} \leq [Si_2O^+]_{BL}/[SiC_3H_9^+]_{BL} \leq a2_{BL}$, wherein $a1_{BL}$ is 1, wherein $a2_{BL}$ is $1*10^5$, wherein $[SiC_3H_9^+]_{BL}$ are the counts of $[SiC_3H_9^+]$ ions in the barrier layer, measured by a TOF-SIMS, and wherein $[Si_2O^+]_{BL}$ are the counts of $[Si_2O^+]$ ions in the barrier layer, measured by a TOF-SIMS, and/or

wherein the protection layer satisfies the condition $a1_{PL} \leq [Si_2O^+]_{PL}/[SiC_3H_9^+]_{PL} \leq a2_{PL}$, wherein $a1_{PL}$ is 1.0, wherein $a2_{PL}$ is $1*10^{10}$, wherein $[SiC_3H_9^+]_{PL}$ are the counts of $[SiC_3H_9^+]$ ions in the protection layer, measured by a TOF-SIMS; and wherein $[Si_2O^+]_{PL}$ are the counts of $[Si_2O^+]$ ions in the protection layer, measured by a TOF-SIMS,

wherein the coated element optionally has a gliding force of 0.1 to 10.0 N, and/or a breaking force of 1 N to 18 N.

In a further aspect, this disclosure provides a coated element having a surface, wherein at least a part of the surface of the element is coated by a coating, wherein the coating comprises, in this order:

- a smoothing layer (SL);

- a barrier layer (BL); and

- a protection layer (PL),

wherein the smoothing layer has a thickness of 1 nm or more, and/or

wherein the barrier layer has a thickness of 1 nm or more, and/or

wherein the protection layer has a gliding force of 0.1 to 10.0 N, and/or a breaking force of 1 N to 18 N, and/or

wherein the coated element has a gliding force of 0.1 to 10.0 N, and/or a breaking force of 1 N to 18 N.

In one embodiment, the coated element further comprising a gliding layer (GL),

wherein the gliding layer is adjacent to the protection layer,

wherein the gliding layer satisfies the condition $a1_{GL} \leq [SiC_3H_9^+]_{GL}/[Si_2O^+]_{GL} \leq a2_{GL}$, wherein $a1_{GL}$ is 1.1, wherein $a2_{GL}$ is $1*10^{10}$, wherein $[SiC_3H_9^+]_{GL}$ are the counts of $[SiC_3H_9^+]$ ions in the gliding layer, measured by a TOF-SIMS; and wherein $[Si_2O^+]_{GL}$ are the counts of $[Si_2O^+]$ ions in the gliding layer, measured by a TOF-SIMS.

In one embodiment, the coated element comprises, preferably consists of glass or polymer, preferably polymer, more preferably cyclic olefin copolymer (COC) and/or cyclic olefin polymer (COP) and/or polypropylene (PP), more preferably cyclic olefin copolymer (COC).

In one embodiment of the coated element, the gliding layer satisfies one or more of the following parameters:

- $a1_{GL} \leq [SiC_3H_9^+]_{GL}/[Si_2O^+]_{GL} \leq a2_{GL}$, wherein $a1_{GL}$ is 1.1, wherein $a2_{GL}$ is $1*10^{10}$, wherein $[SiC_3H_9^+]_{GL}$ are the counts of $[SiC_3H_9^+]$ ions in the gliding layer, measured by a TOF-SIMS; and wherein $[Si_2O^+]_{GL}$ are the counts of $[Si_2O^+]$ ions in the gliding layer, measured by a TOF-SIMS, and

- $b1_{GL} \leq [SiC_2H^+]_{GL}/[SiO^+]_{GL} \leq b2_{GL}$, wherein $b1_{GL}$ is 1.1, wherein $b2_{GL}$ is 1000, wherein $[SiC_2H^+]_{GL}$ are the counts of $[SiC_2H^+]$ ions in the gliding layer, measured by a TOF-SIMS; and wherein $[SiO^+]_{GL}$ are the counts of $[SiO^+]$ ions in the gliding layer, measured by a TOF-SIMS.

In one embodiment of the coated element, the protection layer satisfies one or more of the following parameters:

- $a1_{PL} \leq [Si_2O^+]_{PL}/[SiC_3H_9^+]_{PL} \leq a2_{PL}$, wherein $a1_{PL}$ is 1.0, wherein $a2_{PL}$ is $1*10^{10}$, wherein $[SiC_3H_9^+]_{PL}$ are the counts of $[SiC_3H_9^+]$ ions in the protection layer, measured by a TOF-SIMS; and wherein $[Si_2O^+]_{PL}$ are the counts of $[Si_2O^+]$ ions in the protection layer, measured by a TOF-SIMS, and

- $b1_{PL} \leq [SiC_2H^+]_{PL}/[SiO^+]_{PL} \leq b2_{PL}$, wherein $b1_{PL}$ is 0.5, wherein $b2_{PL}$ is $1*10^5$, wherein $[SiC_2H^+]_{PL}$ are the counts of $[SiC_2H^+]$ ions in the protection layer, measured by a TOF-SIMS; and wherein $[SiO^+]_{PL}$ are the counts of $[SiO^+]$ ions in the protection layer, measured by a TOF-SIMS.

In one embodiment of the coated element, the barrier layer satisfies one or more of the following parameters:

- $a1_{BL} \leq [Si_2O^+]_{BL}/[SiC_3H_9^+]_{BL} \leq a2_{BL}$, wherein $a1_{BL}$ is 1, wherein $a2_{BL}$ is $1*10^5$, wherein $[SiC_3H_9^+]_{BL}$ are the counts of $[SiC_3H_9^+]$ ions in the barrier layer, measured by a TOF-SIMS, and wherein $[Si_2O^+]_{BL}$ are the counts of $[Si_2O^+]$ ions in the barrier layer, measured by a TOF-SIMS, and

- $b1_{BL} \leq [SiO^+]_{BL}/[SiC_2H^+]_{BL} \leq b2_{BL}$, wherein $b1_{BL}$ is 1, wherein $b2_{BL}$ is $1*10^5$, wherein $[SiC_2H^+]_{BL}$ are the counts of $[SiC_2H^+]$ ions in the barrier layer, measured by a TOF-SIMS, wherein $[SiO^+]_{BL}$ are the counts of $[SiO^+]$ ions in the barrier layer, measured by a TOF-SIMS.

In one embodiment of the coated element, the smoothing layer satisfies one or more of the following parameters:

- $a1_{SL} \leq [Si_2O^+]_{SL}/[SiC_3H_9^+]_{SL} \leq a2_{SL}$, wherein $a1_{SL}$ is 1.0, wherein $a2_{SL}$ is $1*10^5$, wherein $[SiC_3H_9^+]_{SL}$ are the counts of $[SiC_3H_9^+]$ ions in the smoothing layer, measured by a TOF-SIMS, wherein $[Si_2O^+]_{SL}$ are the counts of $[Si_2O^+]$ ions in the smoothing layer, measured by a TOF-SIMS, and

- $b1_{SL} \leq [SiC_2H^+]_{SL}/[SiO^+]_{SL} \leq b2_{SL}$, wherein $b1_{SL}$ is 1.0, wherein $b2_{SL}$ is $1*10^5$, wherein $[SiC_2H^+]_{SL}$ are the counts of $[SiC_2H^+]$ ions in the smoothing layer, measured by a TOF-SIMS, wherein $[SiO^+]_{SL}$ are the counts of $[SiO^+]$ ions in the smoothing layer, measured by a TOF-SIMS.

In one embodiment of the coated element, the thickness of the smoothing layer is 1 nm to 25000 nm, or 2 nm to 10000 nm, or 5 nm to 5000 nm, or 100 nm to 2000 nm, or 150 nm to 1500 nm, or 200 nm to 1000 nm, or 250 nm to 500 nm.
In one embodiment of the coated element, the time a sputter gun needs to reach the surface of the element is 0.5 minutes to 150 minutes, or 30 to 120 minutes, or 50 to 100 minutes; and/or wherein in the case positive ions are measured, the point, when the sputter gun reaches the surface of the element is the point, when the counts of $[Si_2O^+]$ ions are equal to the counts of $[C_3H_5^+]$ ions; and/or wherein in the case negative ions are measured, the point, when the sputter gun reaches the surface of the element is the point, when the counts of $[C_4H_3^-]$ ions are equal to the counts of $[SiO_3^-]$ ions; and/or wherein the counts of the ions, preferably the positive and negative ions are obtainable by the method described in the description.
In one embodiment of the coated element, the element is a pharmaceutical container comprising an inner surface, wherein the inner surface is coated, wherein the smoothing layer is in direct contact with the coated element, wherein the gliding layer, if present, is the outermost layer.
In one embodiment of the coated element, one or more of the following conditions is/are fulfilled:

$$[SiO_3^-]_{GL}<[SiO_3^-]_{BL};$$

$$[SiO_3^-]_{PL}<[SiO_3^-]_{BL};$$

$$[SiO_3^-]_{SL}<[SiO_3^-]_{BL}; \text{ and/or}$$

$$[SiO_3^-]_{GL}<[SiO_3^-]_{PL}; \text{ and}$$

wherein $[SiO_3^-]_{GL}$ are the counts of $[SiO_3^-]$ ions in the gliding layer, measured by a TOF-SIMS;

wherein $[SiO_3^-]_{PL}$ are the counts of $[SiO_3^-]$ ions in the protection layer, measured by a TOF-SIMS;

wherein $[SiO_3^-]_{BL}$ are the counts of $[SiO_3^-]$ ions in the barrier layer, measured by a TOF-SIMS;

wherein $[SiO_3^-]_{SL}$ are the counts of $[SiO_3^-]$ ions in the smoothing layer, measured by a TOF-SIMS.

In one aspect, this disclosure provides a system or kit comprising the coated element according to this disclosure, wherein the element is a syringe or cartridge; a plunger, preferably a plunger rod; and optionally a closure, preferably a tip cap and/or a needle shield.

In one aspect, this disclosure provides a closed system comprising the coated element according to this disclosure, wherein the element is a syringe or cartridge; a plunger, preferably a plunger rod; and a closure, preferably a tip cap and/or a needle shield, wherein the closed coated element passes the container closure integrity test according to DIN EN ISO 8871-5:2016; chapter 4.4 in combination with Annex D.

In one embodiment, the closed system comprises a composition, preferably a pharmaceutical composition, preferably a composition containing biologics or mRNA.

In one aspect, this disclosure provides a method for coating an element, preferably a coated element according to this disclosure, comprising the steps:

(1) providing an element comprising a surface;

(2) applying a smoothing layer;

(3) applying a barrier layer;

(4) applying a protection layer; and

(5) optionally applying a gliding layer,

wherein each of the steps (2) to (4) comprises surrounding at least part of the surface of the element with a precursor, and

wherein each of the steps (2) to (4) comprises irradiating the precursor to generate a plasma using a PICVD method,

wherein the step (3) comprises establishing a flow rate of $O_2$ of 10 sccm or more during the irradiation of the precursor.

**Gliding layer**

In one embodiment of the coated element, the gliding layer fulfills the following parameter:

$$a1_{GL} \le [SiC_3H_9^+]_{GL}/[Si_2O^+]_{GL} \le a2_{GL};$$

wherein $a1_{GL}$ is 1.1, preferably 2, more preferably 5, more preferably 10, more preferably 20, more preferably 50, more preferably 100, more preferably 200, more preferably 300, more preferably 400;

wherein $a2_{GL}$ is $1*10^{10}$, preferably $1*10^5$, more preferably 1000, more preferably 800, more preferably 600, more preferably 500;

wherein $[SiC_3H_9^+]_{GL}$ are the counts of $[SiC_3H_9^+]$ ions in the gliding layer, measured by a TOF-SIMS; and

wherein $[Si_2O^+]_{GL}$ are the counts of $[Si_2O^+]$ ions in the gliding layer, measured by a TOF-SIMS.

In one embodiment, $a1_{GL}$ is 1.1, preferably 2, more preferably 5, more preferably 10, more preferably 20, more preferably 50, more preferably 100, more preferably 200, more preferably 300, more preferably 400. In one embodiment, $a2_{GL}$ is $1*10^{10}$, preferably $1*10^5$, more preferably 1000, more preferably 800, more preferably 600, more preferably 500. In one embodiment, $a1_{GL}$ is 1.1, and $a2_{GL}$ is $1*10^{10}$. In one embodiment, $a1_{GL}$ is 10, and $a2_{GL}$ is 1000.

In one embodiment of the coated element, the gliding layer fulfills the following parameter:

$$b1_{GL} \le [SiC_2H^+]_{GL}/[SiO^+]_{GL} \le b2_{GL};$$

wherein $b1_{GL}$ is 1.1, preferably 1.2, more preferably 1.5, more preferably 1.8, more preferably 2.0, more preferably 2.3;

wherein $b2_{GL}$ is 1000, preferably 100, more preferably 10, more preferably 5, more preferably 4, more preferably 3, more preferably 2.5;

wherein $[SiC_2H^+]_{GL}$ are the counts of $[SiC_2H^+]$ ions in the gliding layer, measured by a TOF-SIMS; and

wherein $[SiO^+]_{GL}$ are the counts of $[SiO^+]$ ions in the gliding layer, measured by a TOF-SIMS.

In one embodiment, $b1_{GL}$ is 1.1, preferably 1.2, more preferably 1.5, more preferably 1.8, more preferably 2.0, more preferably 2.3. In one embodiment, $b2_{GL}$ is 1000, preferably 100, more preferably 10, more preferably 5, more preferably 4, more preferably 3, more preferably 2.5. In one embodiment, $b1_{GL}$ is 1.1, and $b2_{GL}$ is 1000. In one embodiment, $b1_{GL}$ is 2.3, and $b2_{GL}$ is 2.5.

In one embodiment of the coated element, the gliding layer fulfills the following parameter:

$$c1_{GL} \leq [SiC_3H_9^+]_{GL}/[SiO^+]_{GL} \leq c1_{GL};$$

wherein $c1_{GL}$ is 1.1, preferably 2, more preferably 5, more preferably 10, more preferably 20, more preferably 50, more preferably 100, more preferably 200, more preferably 300, more preferably 400;

wherein $c2_{GL}$ is $1*10^{10}$, preferably $1*10^5$, more preferably 1000, more preferably 800, more preferably 600, more preferably 500;

wherein $[SiC_3H_9^+]_{GL}$ are the counts of $[SiC_3H_9^+]$ ions in the gliding layer, measured by a TOF-SIMS; and

wherein $[SiO^+]_{GL}$ are the counts of $[SiO^+]$ ions in the gliding layer, measured by a TOF-SIMS.

In one embodiment, $c1_{GL}$ is 1.1, preferably 2, more preferably 5, more preferably 10, more preferably 20, more preferably 50, more preferably 100, more preferably 200, more preferably 300, more preferably 400. In one embodiment, $c2_{GL}$ is $1*10^{10}$, preferably $1*10^5$, more preferably 1000, more preferably 800, more preferably 600, more preferably 500. In one embodiment, $c1_{GL}$ is 1.1, and $c2_{GL}$ is $1*10^{10}$. In one embodiment, $c1_{GL}$ is 400, and $c2_{GL}$ is 500.

In one embodiment of the coated element, the gliding layer fulfills the following parameter:

$$d1_{GL} \leq [SiCH_3O^-]_{GL}/[SiO_3^-]_{GL} \leq d1_{GL};$$

wherein $d1_{GL}$ is 1.0, preferably 1.1, more preferably 1.5, more preferably 2, more preferably 3, more preferably 5, more preferably 8, more preferably 10, more preferably 15, more preferably 20;

wherein $d2_{GL}$ is $1*10^{10}$, preferably $1*10^5$, more preferably 1000, more preferably 200, more preferably 100, more preferably 50, more preferably 30;

wherein $[SiCH_3O^-]_{GL}$ are the counts of $[SiCH_3O^-]$ ions in the gliding layer, measured by a TOF-SIMS; and

wherein $[SiO_3^-]_{GL}$ are the counts of $[SiO_3^-]$ ions in the gliding layer, measured by a TOF-SIMS.

In one embodiment, $d1_{GL}$ is 1.0, preferably 1.1, more preferably 1.5, more preferably 2, more preferably 3, more preferably 5, more preferably 8, more preferably 10, more preferably 15, more preferably 20. In one embodiment, $d2_{GL}$ is $1*10^{10}$, preferably $1*10^5$, more preferably 1000, more preferably 200, more preferably 100, more preferably 50, more preferably 30. In one embodiment, $d1_{GL}$ is 1.0, and $d2_{GL}$ is $1*10^{10}$. In one embodiment, $d1_{GL}$ is 20, and $d2_{GL}$ is 30.

In one embodiment of the coated element, the gliding layer fulfills the following parameter:

$$e1_{GL} \leq [SiCH_3O_2^-]_{GL}/[SiO_3^-]_{GL} \leq e1_{GL};$$

wherein $e1_{GL}$ is 1.0, preferably 1.1, more preferably 1.5, more preferably 2, more preferably 3, more preferably 5, more preferably 8, more preferably 10, more preferably 15, more preferably 20;

wherein $e2_{GL}$ is $1*10^{10}$, preferably $1*10^5$, more preferably 1000, more preferably 200, more preferably 100, more preferably 50, more preferably 30;

wherein $[SiCH_3O_2^-]_{GL}$ are the counts of $[SiCH_3O_2^-]$ ions in the gliding layer, measured by a TOF-SIMS; and

wherein $[SiO_3^-]_{GL}$ are the counts of $[SiO_3^-]$ ions in the gliding layer, measured by a TOF-SIMS.

In one embodiment, $e1_{GL}$ is 1.0, preferably 1.1, more preferably 1.5, more preferably 2, more preferably 3, more preferably 5, more preferably 8, more preferably 10, more preferably 15, more preferably 20. In one embodiment,

$e2_{GL}$ is $1*10^{10}$, preferably $1*10^5$, more preferably 1000, more preferably 200, more preferably 100, more preferably 50, more preferably 30. In one embodiment, $e1_{GL}$ is 1.0, and $e2_{GL}$ is $1*10^{10}$. In one embodiment, $e1_{GL}$ is 20, and $e2_{GL}$ is 30.

In one embodiment of the coated element, the thickness of the gliding layer is 10 nm to 10000 nm, or 15 nm to 5000 nm, or 20 nm to 2000 nm, or 30 nm to 1000 nm, or 40 nm to 500 nm, or 50 nm to 200 nm.

**Protection layer**

In one embodiment of the coated element, the protection layer comprises silicone, preferably:

i) linear silicone; preferably linear polydimethylsiloxane, more preferably hexamethyldisiloxane (HMDSO), octamethyltrisiloxane, decamethyltetrasiloxane; and/or

ii) cyclic silicone; preferably octamethylcyclotetrasiloxane, decamethylcyclopentasiloxane, dodecamethylcyclohexasiloxane, tetramethylcyclotetrasiloxane, pentamethylcyclopentasiloxane; and/or

iii) branched siloxane; and/or

iv) a copolymer comprising any of i) to iii).

In one embodiment of the coated element, the protection layer fulfills the following parameter:

$$a1_{PL} \leq [Si_2O^+]_{PL}/[SiC_3H_9^+]_{PL} \leq a2_{PL};$$

wherein $a1_{PL}$ is 1.0, preferably 1.1, more preferably 1.2, more preferably 1.3, more preferably 1.4, more preferably 1.5, more preferably 2.0, more preferably 3.0;

wherein $a2_{PL}$ is $1*10^{10}$, preferably $1*10^5$, more preferably 100, more preferably 20, more preferably 15, more preferably 10, more preferably 7;

wherein $[SiC_3H_9^+]_{PL}$ are the counts of $[SiC_3H_9^+]$ ions in the protection layer, measured by a TOF-SIMS; and

wherein $[Si_2O^+]_{PL}$ are the counts of $[Si_2O^+]$ ions in the protection layer, measured by a TOF-SIMS.

In one embodiment, $a1_{PL}$ is 1.0, preferably 1.1, more preferably 1.2, more preferably 1.3, more preferably 1.4, more preferably 1.5, more preferably 2.0, more preferably 3.0. In one embodiment, $a2_{PL}$ is $1*10^{10}$, preferably $1*10^5$, more preferably 100, more preferably 20, more preferably 15, more preferably 10, more preferably 7. In one embodiment, $a1_{PL}$ is 1.0, and $a2_{PL}$ is $1*10^{10}$. In one embodiment, $a1_{PL}$ is 3.0, and $a2_{PL}$ is 7.

In one embodiment of the coated element, the protection layer fulfills the following parameter:

$$b1_{PL} \leq [SiC_2H^+]_{PL}/[SiO^+]_{PL} \leq b2_{PL};$$

wherein $[SiC_2H^+]_{PL}$ are the counts of $[SiC_2H^+]$ ions in the protection layer, measured by a TOF-SIMS; and

wherein $[SiO^+]_{PL}$ are the counts of $[SiO^+]$ ions in the protection layer, measured by a TOF-SIMS; and

wherein $b1_{PL}$ is 0.5, preferably 0.8, more preferably 1.0, more preferably 1.1, more preferably 1.2; and

wherein $b2_{PL}$ is $1*10^5$, preferably 100, more preferably 25, more preferably 15, more preferably 5, more preferably 2, more preferably 1.5.

In one embodiment, $b1_{PL}$ is 0.5, preferably 0.8, more preferably 1.0, more preferably 1.1, more preferably 1.2. In one embodiment, $b2_{PL}$ is $1*10^5$, preferably 100, more preferably 25, more preferably 15, more preferably 5, more preferably 2, more preferably 1.5. In one embodiment, $b1_{PL}$ is 0.5, and $b2_{PL}$ is $1*10^5$. In one embodiment, $b1_{PL}$ is 1.2, and $b2_{PL}$ is 1.5.

In one embodiment of the coated element, the protection layer fulfills the following parameter:

$$c1_{PL} \leq [SiO^+]_{PL}/[SiC_3H_9^+]_{PL} \leq c1_{PL};$$

wherein $c1_{PL}$ is 0.5, preferably 0.8, more preferably 1.0, more preferably 1.5, more preferably 2.0, more preferably 2.0, more preferably 2.1;

wherein $c2_{PL}$ is $1*10^5$, preferably 100, more preferably 25, more preferably 20, more preferably 15, more preferably 4, more preferably 2.5;

wherein $[SiC_3H_9^+]_{PL}$ are the counts of $[SiC_3H_9^+]$ ions in the protection layer, measured by a TOF-SIMS; and

wherein $[SiO^+]_{PL}$ are the counts of $[SiO^+]$ ions in the protection layer, measured by a TOF-SIMS.

In one embodiment, $c1_{PL}$ is 0.5, preferably 0.8, more preferably 1.0, more preferably 1.5, more preferably 2.0, more preferably 2.0, more preferably 2.1. In one embodiment, $c2_{PL}$ is $1*10^5$, preferably 100, more preferably 25, more preferably 20, more preferably 15, more preferably 4, more preferably 2.5. In one embodiment, $c1_{PL}$ is 2.1, and $c2_{PL}$ is 2.5. In one embodiment, $c1_{PL}$ is 0.5, and $c2_{PL}$ is $1*10^5$.

In one embodiment of the coated element, the thickness of the protection layer is 1 nm to 8000 nm, or 5 nm to 5000 nm, or 10 nm to 2000 nm, or 20 nm to 1000 nm, or 30 nm to 500 nm, or 50 nm to 300 nm.

**Barrier layer**

In one embodiment of the coated element, the barrier layer fulfills the following parameter:

$$a1_{BL} \leq [Si_2O^+]_{BL}/[SiC_3H_9^+]_{BL} \leq a2_{BL};$$

wherein $a1_{BL}$ is 1, preferably 2, more preferably 4, more preferably 10, more preferably 15, more preferably 20, more preferably 25; and

wherein $a2_{BL}$ is $1*10^5$, preferably 1000, more preferably 500, more preferably 250, more preferably 150, more preferably 100, more preferably 50;

wherein $[SiC_3H_9^+]_{BL}$ are the counts of $[SiC_3H_9^+]$ ions in the barrier layer, measured by a TOF-SIMS; and

wherein $[Si_2O^+]_{BL}$ are the counts of $[Si_2O^+]$ ions in the barrier layer, measured by a TOF-SIMS.

In one embodiment, $a1_{BL}$ is 1, preferably 2, more preferably 4, more preferably 10, more preferably 15, more preferably 20, more preferably 25. In one embodiment, $a2_{BL}$ is $1*10^5$, preferably 1000, more preferably 500, more preferably 250, more preferably 150, more preferably 100, more preferably 50. In one embodiment, $a1_{BL}$ is 1, and $a2_{BL}$ is $1*10^5$. In one embodiment, $a1_{BL}$ is 25, and $a2_{BL}$ is 50.

In one embodiment of the coated element, the barrier layer fulfills the following parameter:

$$b1_{BL} \leq [SiO^+]_{BL}/[SiC_2H^+]_{BL} \leq b2_{BL};$$

wherein $b1_{BL}$ is 1, preferably 5, more preferably 10, more preferably 20, more preferably 30, more preferably 40, more preferably 45; and

wherein $b2_{BL}$ is $1*10^5$, preferably 1000, more preferably 500, more preferably 250, more preferably 150, more preferably 100, more preferably 80;

wherein $[SiC_2H^+]_{BL}$ are the counts of $[SiC_2H^+]$ ions in the barrier layer, measured by a TOF-SIMS; and

wherein $[SiO^+]_{BL}$ are the counts of $[SiO^+]$ ions in the barrier layer, measured by a TOF-SIMS.

In one embodiment, $b1_{BL}$ is 1, preferably 5, more preferably 10, more preferably 20, more preferably 30, more preferably 40, more preferably 45. In one embodiment, $b2_{BL}$ is $1*10^5$, preferably 1000, more preferably 500, more preferably 250, more preferably 150, more preferably 100, more preferably 80. In one embodiment, $b1_{BL}$ is 1, and $b2_{BL}$ is $1*10^5$. In one embodiment, $b1_{BL}$ is 45, and $b2_{BL}$ is 80.

In one embodiment of the coated element, the barrier layer fulfills the following parameter:

$$c1_{BL} \leq [SiO^+]_{BL}/[SiC_3H_9^+]_{BL} \leq c1_{BL};$$

wherein $c1_{BL}$ is 1, preferably 5, more preferably 10, more preferably 50, more preferably 75, more preferably 100, more preferably 110; and

wherein $c2_{BL}$ is $1*10^5$, preferably 1000, more preferably 500, more preferably 400, more preferably 300, more preferably 200, more preferably 150;

wherein $[SiC_3H_9^+]_{BL}$ are the counts of $[SiC_3H_9^+]$ ions in the barrier layer, measured by a TOF-SIMS; and

wherein $[SiO^+]_{BL}$ are the counts of $[SiO^+]$ ions in the barrier layer, measured by a TOF-SIMS.

In one embodiment, $c1_{BL}$ is 1, preferably 5, more preferably 10, more preferably 50, more preferably 75, more preferably 100, more preferably 110. In one embodiment, $c2_{BL}$ is $1*10^5$, preferably 1000, more preferably 500, more preferably 400, more preferably 300, more preferably 200, more preferably 150. In one embodiment, $c1_{BL}$ is 1, and $c2_{BL}$ is $1*10^5$. In one embodiment, $c1_{BL}$ is 110, and $c2_{BL}$ is 150.
In one embodiment of the coated element, the thickness of the barrier layer is 1 nm to 1000 nm, or 5 nm to 500 nm, or 10 nm to 200 nm, or 20 nm to 150 nm.

**Smoothing layer**

In one embodiment of the coated element, the smoothing layer fulfills the following parameter:

$$a1_{SL} \le [Si_2O^+]_{SL}/[SiC_3H_9^+]_{SL} \le a2_{SL};$$

wherein $a1_{SL}$ is 1.0, preferably 2.0, preferably 4.0, more preferably 6.0, more preferably 8.0, more preferably 9.0, more preferably 10.0;

wherein $a2_{SL}$ is $1*10^5$, preferably 1000, preferably 500, more preferably 250, more preferably 100, more preferably 50, more preferably 25, more preferably 15;

wherein $[SiC_3H_9^+]_{SL}$ are the counts of $[SiC_3H_9^+]$ ions in the smoothing layer, measured by a TOF-SIMS; and

wherein $[Si_2O^+]_{SL}$ are the counts of $[Si_2O^+]$ ions in the smoothing layer, measured by a TOF-SIMS.

In one embodiment, $a1_{SL}$ is 1.0, preferably 2.0, preferably 4.0, more preferably 6.0, more preferably 8.0, more preferably 9.0, more preferably 10.0. In one embodiment, $a2_{SL}$ is $1*10^5$, preferably 1000, preferably 500, more preferably 250, more preferably 100, more preferably 50, more preferably 25, more preferably 15. In one embodiment, $a1_{SL}$ is 1.0, and $a2_{SL}$ is $1*10^5$. In one embodiment, $a1_{SL}$ is 10.0, and $a2_{SL}$ is 15.
In one embodiment of the coated element, the smoothing layer fulfills the following parameter:

$$b1_{SL} \le [SiC_2H^+]_{SL}/[SiO^+]_{SL} \le b2_{SL};$$

wherein $[SiC_2H^+]_{SL}$ are the counts of $[SiC_2H^+]$ ions in the smoothing layer, measured by a TOF-SIMS; and

wherein $[SiO^+]_{SL}$ are the counts of $[SiO^+]$ ions in the smoothing layer, measured by a TOF-SIMS; and

wherein $b1_{SL}$ is 1.0, preferably 2.0, more preferably 2.5, more preferably 3.0, more preferably 3.5, more preferably 4.0;

wherein $b2_{SL}$ is $1*10^5$, preferably 100, more preferably 25, more preferably 20, more preferably 15, more preferably 9, more preferably 7.

In one embodiment, $b1_{SL}$ is 1.0, preferably 2.0, more preferably 2.5, more preferably 3.0, more preferably 3.5, more preferably 4.0. In one embodiment, $b2_{SL}$ is $1*10^5$, preferably 100, more preferably 25, more preferably 20, more preferably 15, more preferably 9, more preferably 7. In one embodiment, $b1_{SL}$ is 1.0, and $b2_{SL}$ is $1*10^5$. In one embodiment, $b1_{SL}$ is 4.0, and $b2_{SL}$ is 7.
In one embodiment of the coated element, the smoothing layer fulfills the following parameter:

$$c1_{SL} \le [SiO^+]_{SL}/[SiC_3H_9^+]_{SL} \le c1_{SL};$$

wherein $c1_{SL}$ is 0.5, preferably 0.8, more preferably 1.0, more preferably 1.5, more preferably 2.0, more preferably 2.0, more preferably 2.1;

wherein $c2_{PL}$ $1*10^5$, preferably 100, more preferably 25, more preferably 20, more preferably 15, more preferably 4, more preferably 2.5;

wherein $[SiC_3H_9^+]_{SL}$ are the counts of $[SiC_3H_9^+]$ ions in the smoothing layer, measured by a TOF-SIMS; and

wherein $[SiO^+]_{SL}$ are the counts of $[SiO^+]$ ions in the smoothing layer, measured by a TOF-SIMS.

In one embodiment, $c1_{SL}$ is 0.5, preferably 0.8, more preferably 1.0, more preferably 1.5, more preferably 2.0, more preferably 2.0, more preferably 2.1. In one embodiment, $c2_{PL}$ $1*10^5$, preferably 100, more preferably 25, more preferably 20, more preferably 15, more preferably 4, more preferably 2.5. In one embodiment, $c1_{SL}$ is 0.5, and $c2_{PL}$ $1*10^5$. In one embodiment, $c1_{SL}$ is 2.1, and $c2_{PL}$ 2.5.

In one embodiment of the coated element, the smoothing layer fulfills the following parameter:

$$x1_{SL} \le [SiC_2H^+]_{SL}/[C_3H_5^+]_{SL} \le x2_{SL};$$

wherein $[SiC_2H^+]_{SL}$ are the counts of $[SiC_2H^+]$ ions in the smoothing layer, measured by a TOF-SIMS; and

wherein $[C_3H_5^+]_{SL}$ are the counts of $[C_3H_5^+]$ ions in the smoothing layer, measured by a TOF-SIMS; and

wherein $x1_{SL}$ is 1, preferably 2, more preferably 10, more preferably 50, more preferably 100; and

wherein $x2_{SL}$ is $1*10^{10}$, preferably $1*10^5$, more preferably $1*10^4$, more preferably $1*10^3$.

In one embodiment, $x1_{SL}$ is 1, preferably 2, more preferably 10, more preferably 50, more preferably 100. In one embodiment, $x2_{SL}$ is $1*10^{10}$, preferably $1*10^5$, more preferably $1*10^4$, more preferably $1*10^3$. In one embodiment, $x1_{SL}$ is 1, and $x2_{SL}$ is $1*10^{10}$. In one embodiment, $x1_{SL}$ is 100, and $x2_{SL}$ is $1*10^3$.

In one embodiment of the coated element, the smoothing layer fulfills the following parameter(s):

$$y1_{SL} \le [Si_2O^+]_{SL}/[C_3H_5^+]_{SL} \le y2_{SL};$$

wherein $[Si_2O^+]_{SL}$ are the counts of $[Si_2O^+]$ ions in the smoothing layer, measured by a TOF-SIMS;

wherein $[C_3H_5^+]_{SL}$ are the counts of $[C_3H_5^+]$ ions in the smoothing layer, measured by a TOF-SIMS;

wherein $y1_{SL}$ is 1; preferably 5, more preferably 20, more preferably 50, more preferably 100; and

wherein $y2_{SL}$ is $1*10^{10}$, preferably $1*10^5$, more preferably $1*10^4$, more preferably $1*10^3$.

In one embodiment, $y1_{SL}$ is 1; preferably 5, more preferably 20, more preferably 50, more preferably 100. In one embodiment, $y2_{SL}$ is $1*10^{10}$, preferably $1*10^5$, more preferably $1*10^4$, more preferably $1*10^3$. In one embodiment, $y1_{SL}$ is 1, and $y2_{SL}$ is $1*10^{10}$. In one embodiment, $y1_{SL}$ is 100, and $y2_{SL}$ is $1*10^3$.

In one embodiment of the coated element, the smoothing layer fulfills the following parameter(s):

$$z1_{SL} \le [SiO^+]_{SL}/[C_3H_5^+]_{SL} \le z2_{SL};$$

wherein $[SiO^+]_{SL}$ are the counts of $[SiO^+]$ ions in the smoothing layer, measured by a TOF-SIMS;

wherein $[C_3H_5^+]_{SL}$ are the counts of $[C_3H_5^+]$ ions in the smoothing layer, measured by a TOF-SIMS;

wherein $z1_{SL}$ is 1; preferably 5, more preferably 20, more preferably 50, more preferably 70; and

wherein $z2_{SL}$ is $1*10^{10}$, preferably $1*10^5$, more preferably $1*10^4$, more preferably $1*10^3$.

In one embodiment of the coated element, the thickness of the smoothing layer is 1 nm to 20000 nm, preferably 5 nm to 5000, more preferably 100 nm to 2000nm, more preferably 150 nm to 1500nm, more preferably 200 nm to 1000nm, more preferably 250 nm to 500nm.

In one embodiment, $z1_{SL}$ is 1; preferably 5, more preferably 20, more preferably 50, more preferably 70. In one embodiment, $z2_{SL}$ is $1*10^{10}$, preferably $1*10^5$, more preferably $1*10^4$, more preferably $1*10^3$. In one embodiment, $z1_{SL}$ is 1, and $z2_{SL}$ is $1*10^{10}$. In one embodiment, $z1_{SL}$ is 70, and $z2_{SL}$ is $1*10^3$.

**Coated element**

In one embodiment of the coated element, one or both of following equations is fulfilled:

$$R3 \leq t_{SL}/t_{CO};$$

and/or

$$t_{SL}/t_{CO} \leq R4;$$

wherein $t_{SL}$ is the time sputter through the smoothing layer; and

wherein $t_{CO}$ is the time sputter through the coating;

wherein R3 is 0.01, preferably 0.05, more preferably 0.10, more preferably 0.20, more preferably 0.30, more preferably 0.40, more preferably 0.50, more preferably 0.60, more preferably 0.65; and

wherein R4 is 5.00, preferably 4.00, more preferably 3.00, more preferably 2.00, more preferably 1.00, more preferably 0.80, more preferably 0.70.

In one embodiment of the coated element, the time a sputter gun needs to reach the surface of the element is 0.5 minutes to 150 minutes, preferably 30 to 120 minutes, more preferably 50 to 100 minutes; and/or

wherein in the case positive ions are measured, the point, when the sputter gun reaches the surface of the element is the point, when the counts of $[Si_2O^+]$ ions are equal to the counts of $[C_3H_5^+]$ ions; and/or

wherein in the case negative ions are measured, the point, when the sputter gun reaches the surface of the element is the point, when the counts of $[C_4H_3^-]$ ions are equal to the counts of $[SiO_3^-]$ ions; and/or

wherein the counts of the ions, preferably the positive and negative ions are obtainable by the method described in the description.

In one embodiment of the coated element, the at least a part of the element is 10 % to 90%, preferably 20 % to 80 %, more preferably 30 % to 70 %, more preferably 40 % to 60 % of the surface of the element.

In one embodiment of the coated element, the element is a pharmaceutical container comprising an inner surface, and wherein the at least a part of the surface comprises at least a part of the inner surface of the pharmaceutical container, preferably comprises the inner surface of the pharmaceutical container, more preferably is the inner surface of the pharmaceutical container.

In one embodiment of the coated element, the smoothing layer is in direct contact with the element.

In one embodiment of the coated element, the gliding layer is the outermost layer.

In one embodiment of the coated element, the element comprises a partially uncoated surface, and

wherein one or both of following equations is fulfilled:

$$R1 \leq T_{SL}/SRz;$$

and/or

$$T_{SL}/SRz \leq R2;$$

wherein R1 [nm/nm] is 0.005, 0.01, or 0.05, or 0.1, preferably 0.2, more preferably 0.3, more preferably 0.5, more preferably 1.0, more preferably 2.0, more preferably 3.0, more preferably 4.0, more preferably 5.0; and/or

wherein R2 [nm/nm] is 20.0, preferably 18.0, more preferably 15.0, more preferably 12.0, more preferably 10.0, more preferably 8.0, more preferably 6.0;

wherein $T_{SL}$ is the thickness of the smoothing layer in at least a part of the coating; and

wherein SRz is the surface roughness depth of at least a part of the partially uncoated surface of the element.

In one embodiment of the coated element, the roughness SRz of the partially uncoated surface of the element is 1 nm to 5000 nm, 10 nm to 3000 nm, 50 nm to 2000 nm, 70 nm to 1000 nm, or 100 nm to 500 nm. In one embodiment of the coated element, the roughness SRz of the partially uncoated surface of the element is 1 nm or more, 10 nm or more, 50 nm or more, 70 nm or more, or 100 nm or more. In one embodiment of the coated element, the roughness SRz of the partially uncoated surface of the element is 5000 nm or less, 3000 nm or less, 2000 nm or less, 1000 nm or less, or 500 nm or less.

In one embodiment of the coated element, the gliding layer, the protection layer, the barrier layer and/or the smoothing layer comprise(s) $SiO_xC_y$, measured by XPS,

wherein x is from 0.3 to 3.0, preferably 0.5 to 2.0, more preferably 0.9 to 1.5, and/or

wherein y is from 0 to 6.0, preferably 0.5 to 4.0, more preferably 2.5 to 3.5.

In one embodiment of the coated element, one or more of the following conditions is/are fulfilled:

$$[SiO_3^-]_{GL} < [SiO_3^-]_{BL};$$

$$[SiO_3^-]_{PL} < [SiO_3^-]_{BL};$$

$$[SiO_3^-]_{SL} < [SiO_3^-]_{BL}; \text{ and/or}$$

$$[SiO_3^-]_{GL} < [SiO_3^-]_{PL};$$

wherein $[SiO_3^-]_{GL}$ are the counts of $[SiO_3^-]$ ions in the gliding layer, measured by a TOF-SIMS;

wherein $[SiO_3^-]_{PL}$ are the counts of $[SiO_3^-]$ ions in the protection layer, measured by a TOF-SIMS;

wherein $[SiO_3^-]_{BL}$ are the counts of $[SiO_3^-]$ ions in the barrier layer, measured by a TOF-SIMS;

wherein $[SiO_3^-]_{SL}$ are the counts of $[SiO_3^-]$ ions in the smoothing layer, measured by a TOF-SIMS.

In one embodiment of the coated element, one or more of the following conditions is/are fulfilled:

    i)        $[SiC^-]_{PL} > [SiC^-]_{BL};$

    ii)       $[SiC^-]_{PL} < [SIC^-]_{SL}, \text{ or } [SiC^-]_{PL} > [SiC^-]_{SL};$

    iii)      $[SiC^-]_{PL} > [SiC^-]_{GL}; \text{ and/or}$

    iv)      $[SiC^-]_{GL} > [SiC^-]_{BL};$

wherein $[SiC^-]_{GL}$ are the counts of $[SiC^-]$ ions in the gliding layer, measured by a TOF-SIMS;

wherein $[SiC^-]_{PL}$ are the counts of $[SiC^-]$ ions in the protection layer, measured by a TOF-SIMS;

wherein $[SiC^-]_{BL}$ are the counts of $[SiC^-]$ ions in the barrier layer, measured by a TOF-SIMS;

wherein $[SiC^-]_{SL}$ are the counts of $[SiC^-]$ ions in the smoothing layer, measured by a TOF-SIMS.

In one embodiment of the coated element, one or more of the following conditions is/are fulfilled:

$$[SiC_2H^+]_{BL} < [SiC_2H^+]_{SL};$$

$$[SiC_2H^+]_{GL} < [SiC_2H^+]_{SL};$$

$$[SiC_2H^+]_{BL} < [SiC_2H^+]_{PL};$$

$$[SiC_2H^+]_{GL} < [SiC_2H^+]_{PL}; \text{ and/or}$$

$$[SiC_2H^+]_{GL} > [SiC_2H^+]_{BL};$$

wherein $[SiC_2H^+]_{GL}$ are the counts of $[SiC_2H^+]$ ions in the gliding layer, measured by a TOF-SIMS;

wherein $[SiC_2H^+]_{PL}$ are the counts of $[SiC_2H^+]$ ions in the protection layer, measured by a TOF-SIMS;

wherein $[SiC_2H^+]_{BL}$ are the counts of $[SiC_2H^+]$ ions in the barrier layer, measured by a TOF-SIMS;

wherein $[SiC_2H^+]_{SL}$ are the counts of $[SiC_2H^+]$ ions in the smoothing layer, measured by a TOF-SIMS.

In one embodiment of the coated element, one or more of the following conditions is/are fulfilled:

$$[SiO^+]_{GL} < [SiO^+]_{BL};$$

$$[SiO^+]_{PL} < [SiO^+]_{BL};$$

$$[SiO^+]_{SL} < [SiO^+]_{BL}; \text{ and/or}$$

$$[SiO^+]_{GL} < [SiO^+]_{PL};$$

wherein $[SiO^+]_{GL}$ are the counts of $[SiO^+]$ ions in the gliding layer, measured by a TOF-SIMS;

wherein $[SiO^+]_{PL}$ are the counts of $[SiO^+]$ ions in the protection layer, measured by a TOF-SIMS;

wherein $[SiO^+]_{BL}$ are the counts of $[SiO^+]$ ions in the barrier layer, measured by a TOF-SIMS;

wherein $[SIO^+]_{SL}$ are the counts of $[SiO^+]$ ions in the smoothing layer, measured by a TOF-SIMS.

In one embodiment of the coated element, the thickness of the coating is 1 nm to 1 mm, 10 nm to 0.5 mm, 200 nm to 2000 nm, or 500 nm to 1500 nm.

In one embodiment of the coated element, the smoothing layer, the barrier layer, the protection layer and/or the gliding layer, more preferably all layers, comprise(s), preferably consist(s) of, the elements Si, O and C measured by XPS.

In one embodiment of the coated element, the breaking force of the coated element is 1 N to 18 N, preferably 1.5 N to 10 N, or 2.0 N to 7 N. In one embodiment of the coated element, the breaking force of the coated element is 1 N or more, 1.5 N or more, or 2.0 N or more. In one embodiment of the coated element, the breaking force of the coated element is 18 N or less, 10 N or less, 7 N or less, or 5 N or less.

In one embodiment of the coated element, the gliding force of the coated element is 0.1 N to 10.0 N, preferably 0.3 N to 7.0 N, more preferably 0.4 N to 5.0 N, more preferably 0.5 N to 4.0 N, more preferably 1.0 N to 3.0 N. In one embodiment of the coated element, the gliding force of the coated element is 0.1 N or more, 0.3 N or more, 0.4 N or more, 0.5 N or more, or 1.0 N or more. In one embodiment of the coated element, the gliding force of the coated element is 10.0 N or less, 7.0 N or less, 5.0 N or less, 4.0 N or less, or 3.0 N or less.

In one embodiment of the coated element, one or more of the following equation(s) are fulfilled:

BF7/BF1 ≤ bf; and/or

BF28/BF1 ≤ bf; and/or

BF28/BF7 ≤ bf;

wherein BF1 is the breaking force after 1 day,

wherein BF7 is the breaking force after 7 days,

wherein BF28 is the breaking force after 28 days,

wherein bf is 4.0, preferably 3.0, more preferably 2.0, more preferably 1.8, more preferably 1.6, more preferably 1.4, more preferably 1.2.

In one embodiment of the coated element, the smoothing layer, the barrier layer, the protection layer and/or the gliding layer, more preferably all layers, is/are obtainable by the method according to any one of the following items.

**System or kit**

In one aspect a system or a kit is provided, the system or the kit comprising:

the coated element according to this disclosure, wherein the element is a syringe or cartridge, and

a plunger, preferably and a plunger rod;

preferably a closure, more preferably a tip cap and/or a needle shield.

**Closed system**

In one aspect a closed system is provided, the closed system comprising:

the coated element according to this disclosure, wherein the element is a syringe or cartridge, and

a plunger, preferably a plunger rod;

preferably a closure, more preferably a tip cap and/or a needle shield; and wherein the closed coated element passes the container closure integrity test according to DIN EN ISO 8871-5:2016; chapter 4.4 in combination with Annex D.

In one embodiment, the closed system comprises a composition, preferably a pharmaceutical composition, more preferably a composition containing biologics or mRNA.

**Method**

In one aspect a method for coating an element is provided, preferably a method to obtain a coated element according to this disclosure, comprising the steps:

- providing an element comprising a surface;

- applying one or more layers, preferably a smoothing layer, a barrier layer, a protection layer and optionally a gliding layer; by performing a coating process on at least part of the surface, comprising the steps:

a) surrounding the at least part of the surface of the element with a precursor; and

b) irradiating the precursor to generate a plasma.

In one embodiment of the method, the coating process is a plasma-enhanced chemical vapor deposition (PECVD) process, plasma impulse chemical vapor deposition (PICVD) process or plasma assisted chemical vapor deposition (PACVD) process, preferably a plasma impulse chemical vapor deposition (PICVD) process.

In a most preferred embodiment of the method, the coating process is a plasma impulse chemical vapor deposition (PICVD) process.

In one embodiment of the method, the precursor comprises one or more of hexamethyldisiloxane (HMDSO), hexamethyldisilazane (HMDSN), tetramethylsilane (TMS), trimethylborazole (TMB), tri(dimethylaminosilyl)-amino-di(dimethylamino)borane (TDADB), tris(trimethylsilyl)borate (TMSB), hexamethylcyclotrisiloxan (HMCTSO), octamethylcyclotetrasiloxan (OMCTS), decamethylcyclopentasiloxan (DMCPS), dodecamethylcyclohexasiloxan (DMCHS) diac-etoxy-di-t-butoxysilane (DADBS), tetraethoxysilane (TEOS), tris(trimethylsilyloxy)vinylsilane (TTMSVS), vinyltriethoxysilane (VTES) and/or combinations thereof, preferably the precursor is HMDSO.

In one embodiment of the method, the precursor comprises, preferably consists of, the elements Si, C, O and H.

In one embodiment of the method, for the application of the smoothing layer, the barrier layer, the protection layer and/or the gliding layer, one or more of the following conditions is/are fulfilled:

i. wherein the coating process is a plasma impulse chemical vapor deposition (PICVD) process,

ii. wherein the irradiation is carried out by a microwave generator, preferably wherein the ray has a frequency of 300 MHz to 300 GHz, more preferably 600 MHz to 100 GHz, more preferably 800 MHz to 10 GHz, more preferably 900 MHz to 3 GHz, more preferably 2.45 GHz; and/or

iii. wherein the input power IP, preferably the input power IP of the microwave generator, is 100 W to 10000 W, preferably 300 W to 9000 W, more preferably 500 W to 8000 W, more preferably 700 W to 6000 W, more preferably 900 W to 4000 W, more preferably 1100 W to 2000 W; and/or

iv. wherein the precursor P comprises one or more of hexamethyldisiloxane (HMDSO), hexamethyldisilazane (HMDS), tetramethylsilane (TMS), trimethylborazole (TMB), tri(dimethylaminosilyl)-amino-di(dimethylamino)borane (TDADB), tris(trimethylsilyl)borate (TMSB), hexamethylcyclotrisiloxan (HMCTSO), octamethylcyclotetrasiloxan (OMCTS), decamethylcyclopentasiloxan (DMCPS), dodecamethylcyclohexasiloxan (DMCHS) diac-etoxy-di-t-butoxysilane (DADBS), tetraethoxysilane (TEOS), tris(trimethylsilyloxy)vinylsilane (TTMSVS), vinyltriethoxysilane (VTES) and/or combinations thereof, preferably the precursor P is HMDSO; and/or

wherein, besides the precursor P, a gas, preferably oxygen, is fed.

In one embodiment of the method, for the application of the smoothing layer one or more of the following conditions is/are fulfilled:

i. wherein the process temperature $PT_{SL}$ is 15 °C to 150 °C, 20 °C to 80 °C, or 25 °C to 40 °C; and/or

ii. wherein the pulse duration $PD_{SL}$ of the plasma is 0.0001 ms to 1.5 ms, 0.1 ms to 1.2 ms, or preferably 0.3 ms to 0.8 ms; and/or

iii. the pulse pause $PP_{SL}$ between two pulses is 0.01 ms to 100 ms, 0.1 ms to 70 ms, 1.0 ms to 50 ms, or 5.0 ms to 20 ms; and/or

iv. the total time $TT_{SL}$ of irradiation is 1 s to 50 s, 2 s to 20 s, 3 s to 10 s, or 4 s to 7 s; and/or

v. the ratio [ms/ms] of all pulse durations $PD_{SL}$ [ms] to all pulse pauses $PP_{SL}$ [ms] is 0.001 to 1, 0.01 to 0.5, or 0.03 to 0.08; and/or

vi. wherein the process pressure $PR_{SL}$ is 0.1 mbar to 100 mbar, 0.3 mbar to 10 mbar, 0.5 to 3.0 mbar, or 0.5 to 0.8 mbar; and/or

vii. wherein the flow rate of the precursor P is 1 sccm to 200 sccm, 5 sccm to 100 sccm, or 15 sccm to 30 sccm,

wherein, besides the precursor, no further substance (such as a gas) is fed.

In one embodiment of the method, for the application of the barrier layer one or more of the following conditions is/are fulfilled:

i. wherein the process temperature $PT_{BL}$ is 15 °C to 200°C, or 25 °C to 70 °C; and/or

ii. wherein the pulse duration $PD_{BL}$ of the plasma is 0.5 ms to 50 ms, 1.0 to 10 ms, or 1.5 ms to 2.5 ms; and/or

iii. the pulse pause $PP_{BL}$ between two pulses is 0.05 ms to 500 ms, 0.1 ms to 100 ms, 1.0 ms to 30 ms, or 3.0 ms to 10 ms; and/or

iv. the total time $TT_{BL}$ of irradiation is 1 s to 50 s, 3 s to 30 s, 5 s to 15 s, or 6 s to 12 s; and/or

v. the ratio [ms/ms] of all pulse durations $PD_{BL}$ [ms] to all pulse pauses $PP_{BL}$ [ms] is 0.01 to 1, preferably 0.1 to 0.8, more preferably 0.3 to 0.6; and/or

vi. wherein the process pressure $PR_{BL}$ is 0.01 mbar to 10 mbar, 0.1 mbar to 5 mbar, 0.2 mbar to 3.0 mbar, or 0.25 mbar to 0.8 mbar; and/or

vii. wherein the flow rate of the precursor P is 0.01 sccm to 20 sccm, 0.1 sccm to 5.0 sccm, or 0.3 sccm to 0.9 sccm,

wherein, besides the precursor, oxygen gas is fed and the flow rate of the oxygen gas is 0.1 to 100 sccm, 5 sccm to 80 sccm, or 30 sccm to 50 sccm.

In one embodiment of the method, for the application of the protection layer one or more of the following conditions is/are fulfilled:

i. wherein the process temperature $PT_{PL}$ is 15 °C to 150 °C, 30 °C to 100 °C, or 40 °C to 70 °C; and/or

ii. wherein the pulse duration $PD_{PL}$ of the plasma is 0.001 ms to 1.5 ms, 0.01 ms to 1.0 ms, or 0.3 ms to 0.8 ms; and/or

iii. the pulse pause $PP_{PL}$ between two pulses is 0.01 ms to 100 ms, 1 ms to 50 ms, 3 ms to 20 ms, 5 ms to 15 ms, or 8 ms to 12 ms; and/or

iv. the total time $TT_{PL}$ of irradiation is 0.1 s to 20 s, 0.3 s to 10 s, 0.7 s to 8 s, or 0.8 s to 1.2 s; and/or

v. the ratio [ms/ms] of all pulse durations $PD_{PL}$ [ms] to all pulse pauses $PP_{PL}$ [ms] is 0.001 to 1, preferably 0.01 to 0.5, more preferably 0.03 to 0.08; and/or

vi. wherein the process pressure $PR_{PL}$ is 0.1 mbar to 100 mbar, 0.1 mbar to 20 mbar, 0.3 mbar to 1.0 mbar, or 0.5 mbar to 0.8 mbar; and/or

vii. wherein the flow rate of the precursor $P_{PL}$ is 1 sccm to 200 sccm, 5 sccm to 40 sccm, or 10 sccm to 20 sccm,

wherein, besides the precursor, oxygen gas is fed and the flow rate of the gas is 0.1 to 100, preferably 2 sccm to 30 sccm, more preferably 4 sccm to 8 sccm.

In one embodiment of the method, for the application of the gliding layer one or more of the following conditions is/are fulfilled:

i. wherein the process temperature $PT_{GL}$ is 15 °C to 150 °C, 25 °C to 100 °C, or 40 °C to 70 °C; and/or

ii. wherein the pulse duration $PD_{GL}$ of the plasma is 0.001 ms to 1.5 ms, 0.01 ms to 0.8 ms, or 0.02 ms to 0.1 ms; and/or

iii. the pulse pause $PP_{GL}$ between two pulses is 0.001 ms to 100 ms, 0.01 ms to 20 ms, 0.1 ms to 10 ms, 0.3 ms to 3 ms; and/or

iv. the total time $TT_{GL}$ of irradiation is 1 s to 50 s, 3 s to 30 s, 5 s to 15 s, or 6 s to 10 s; and/or

v. the ratio [ms/ms] of all pulse durations $PD_{GL}$ [ms] to all pulse pauses $PP_{GL}$ [ms] is 0.001 to 1, preferably 0.01 to 0.5, more preferably 0.02 to 0.08; and/or

vi. wherein the process pressure $PR_{GL}$ is 0.1 mbar to 100 mbar, 0.2 mbar to 10 mbar, 0.3 to 1.5 mbar, or 0.6 to 1.0 mbar; and/or

vii. wherein the flow rate of the precursor $P_{GL}$ is 1 sccm to 200 sccm, 3 sccm to 50 sccm, or 5 sccm to 15 sccm,

wherein, besides the precursor, no further substance is fed.

**Method (further embodiments)**

In one aspect a method for coating an element is provided, preferably a method to obtain a coated element according to this disclosure, comprising the steps:

- providing an element comprising a surface;

- applying a smoothing layer, a barrier layer, a protection layer and optionally a gliding layer; by performing a coating process on at least part of the surface, comprising the steps:

 a) surrounding the at least part of the surface of the element with a precursor; and
 b) irradiating the precursor to generate a plasma,

wherein the following conditions are fulfilled:

 i. the coating process is a plasma impulse chemical vapor deposition (PICVD) process,

 ii. the irradiation is carried out by a microwave generator, wherein the ray has a frequency of MHz to 3 GHz, more preferably 2.45 GHz; and/or

 iii. the input power IP of the microwave generator is 900 W to 2000 W; and/or

 iv. wherein the precursor P comprises one or more of hexamethyldisiloxane (HMDSO), hexamethyldisilazane (HMDS), tetramethylsilane (TMS), trimethylborazole (TMB), tri(dimethylaminosilyl)-amino-di(dimethylamino)borane (TDADB), tris(trimethylsilyl)borate (TMSB), hexamethylcyclotrisiloxan (HMCTSO), octamethylcyclotetrasiloxan (OMCTS), decamethylcyclopentasiloxan (DMCPS), dodecamethylcyclohexasiloxan (DMCHS) diacetoxy-di-t-butoxysilane (DADBS), tetraethoxysilane (TEOS), tris(trimethylsilyloxy)vinylsilane (TTMSVS), vinyltriethoxysilane (VTES) and/or combinations thereof, preferably the precursor P is HMDSO,

wherein, besides the precursor P, oxygen gas is fed during the application of the barrier layer.

In one embodiment of the method, for the application of the smoothing layer the following conditions are fulfilled:

 i. the process temperature $PT_{SL}$ is 15 °C to 150 °C;

 ii. the pulse duration $PD_{SL}$ of the plasma is 0.0001 ms to 1.5 ms;

 iii. the pulse pause $PP_{SL}$ between two pulses is 0.01 ms to 100 ms;

 iv. the total time $TT_{SL}$ of irradiation is 1 s to 50 s;

 v. the ratio [ms/ms] of all pulse durations $PD_{SL}$ [ms] to all pulse pauses $PP_{SL}$ [ms] is 0.001 to 1;

 vi. the process pressure $PR_{SL}$ is 0.1 mbar to 100 mbar;

 vii. the flow rate of the precursor P is 1 sccm to 200 sccm,

wherein, besides the precursor, no further substance (such as a gas) is fed.

In one embodiment of the method, for the application of the smoothing layer the following conditions are fulfilled:

 i. the process temperature $PT_{SL}$ is 25 °C to 40 °C;

 ii. the pulse duration $PD_{SL}$ of the plasma is 0.3 ms to 0.8 ms;

 iii. the pulse pause $PP_{SL}$ between two pulses is 5.0 ms to 20 ms;

iv. the total time $TT_{SL}$ of irradiation is 4 s to 7 s;

v. the ratio [ms/ms] of all pulse durations $PD_{SL}$ [ms] to all pulse pauses $PP_{SL}$ [ms] is 0.03 to 0.08;

vi. the process pressure $PR_{SL}$ is 0.5 to 0.8 mbar;

vii. the flow rate of the precursor P is 15 sccm to 30 sccm,

wherein, besides the precursor, no further substance (such as a gas) is fed.

In one embodiment of the method, for the application of the barrier layer the following conditions are fulfilled:

i. the process temperature $PT_{BL}$ is 15 °C to 200 °C;

ii. the pulse duration $PD_{BL}$ of the plasma is 0.5 ms to 50 ms;

iii. the pulse pause $PP_{BL}$ between two pulses is 0.05 ms to 500 ms;

iv. the total time $TT_{BL}$ of irradiation is 1 s to 50 s;

v. the ratio [ms/ms] of all pulse durations $PD_{BL}$ [ms] to all pulse pauses $PP_{BL}$ [ms] is 0.01 to 1;

vi. the process pressure $PR_{BL}$ is 0.01 mbar to 10 mbar;

vii. the flow rate of the precursor P is 0.01 sccm to 20 sccm,

wherein, besides the precursor, oxygen gas is fed and the flow rate of the oxygen gas is 0.1 to 100 sccm.

In one embodiment of the method, for the application of the barrier layer the following conditions are fulfilled:

i. the process temperature $PT_{BL}$ is 25 °C to 70 °C;

ii. the pulse duration $PD_{BL}$ of the plasma is 1.5 ms to 2.5 ms;

iii. the pulse pause $PP_{BL}$ between two pulses is 3.0 ms to 10 ms;

iv. the total time $TT_{BL}$ of irradiation is 6 s to 12 s;

v. the ratio [ms/ms] of all pulse durations $PD_{BL}$ [ms] to all pulse pauses $PP_{BL}$ [ms] is 0.3 to 0.6;

vi. the process pressure $PR_{BL}$ is 0.25 mbar to 0.8 mbar;

vii. the flow rate of the precursor P is 0.3 sccm to 0.9 sccm,

wherein, besides the precursor, oxygen gas is fed and the flow rate of the oxygen gas is 30 sccm to 50 sccm.

In one embodiment of the method, for the application of the protection layer the following conditions are fulfilled:

i. the process temperature $PT_{PL}$ is 15 °C to 150 °C;

ii. the pulse duration $PD_{PL}$ of the plasma is 0.001 ms to 1.5 ms;

iii. the pulse pause $PP_{PL}$ between two pulses is 0.01 ms to 100 ms;

iv. the total time $TT_{PL}$ of irradiation is 0.1 s to 20 s;

v. the ratio [ms/ms] of all pulse durations $PD_{PL}$ [ms] to all pulse pauses $PP_{PL}$ [ms] is 0.001 to 1;

vi. the process pressure $PR_{PL}$ is 0.1 mbar to 100 mbar; and

vii. the flow rate of the precursor $P_{PL}$ is 1 sccm to 200 sccm,

wherein, besides the precursor, oxygen gas is fed and the flow rate of the gas is 0.1 to 100 sccm.

In one embodiment of the method, for the application of the protection layer the following conditions are fulfilled:

i. the process temperature $PT_{PL}$ is 40 °C to 70 °C;

ii. the pulse duration $PD_{PL}$ of the plasma is 0.3 ms to 0.8 ms;

iii. the pulse pause $PP_{PL}$ between two pulses is 8 ms to 12 ms;

iv. the total time $TT_{PL}$ of irradiation is 0.8 s to 1.2 s;

v. the ratio [ms/ms] of all pulse durations $PD_{PL}$ [ms] to all pulse pauses $PP_{PL}$ [ms] is 0.03 to 0.08;

vi. the process pressure $PR_{PL}$ is 0.5 mbar to 0.8 mbar; and

vii. the flow rate of the precursor $P_{PL}$ is 10 sccm to 20 sccm,

wherein, besides the precursor, oxygen gas is fed and the flow rate of the gas is 4 sccm to 8 sccm.

In one embodiment of the method, for the application of the gliding layer the following conditions are fulfilled:

i. the process temperature $PT_{GL}$ is 15 °C to 150 °C;

ii. the pulse duration $PD_{GL}$ of the plasma is 0.001 ms to 1.5 ms;

iii. the pulse pause $PP_{GL}$ between two pulses is 0.001 ms to 100 ms;

iv. the total time $TT_{GL}$ of irradiation is 1 s to 50 s;

v. the ratio [ms/ms] of all pulse durations $PD_{GL}$ [ms] to all pulse pauses $PP_{GL}$ [ms] is 0.001 to 1;

vi. the process pressure $PR_{GL}$ is 0.1 mbar to 100 mbar; and

vii. the flow rate of the precursor $P_{GL}$ is 1 sccm to 200 sccm,

wherein, besides the precursor, no further substance is fed.

In one embodiment of the method, for the application of the gliding layer the following conditions are fulfilled:

i. the process temperature $PT_{GL}$ is 40 °C to 70 °C;

ii. the pulse duration $PD_{GL}$ of the plasma is 0.02 ms to 0.1 ms;

iii. the pulse pause $PP_{GL}$ between two pulses is 0.3 ms to 3 ms;

iv. the total time $TT_{GL}$ of irradiation is 6 s to 10 s;

v. the ratio [ms/ms] of all pulse durations $PD_{GL}$ [ms] to all pulse pauses $PP_{GL}$ [ms] is 0.02 to 0.08;

vi. the process pressure $PR_{GL}$ is 0.6 to 1.0 mbar; and

vii. the flow rate of the precursor $P_{GL}$ is 5 sccm to 15 sccm,

wherein, besides the precursor, no further substance is fed.

**Pharmaceutical container**

In one embodiment of the coated element according to this disclosure or the method according to this disclosure, the element is a pharmaceutical container, preferably a vial, ampule, cartridge or syringe, more preferably a cartridge or syringe, most preferably a syringe.

In one embodiment, the element defines a lumen, wherein the brimful volume of the element is 0.1 ml to 200 ml, 0.2 ml to 100 ml, 0.4 ml to 50 ml, 0.6 ml to 20 ml, 0.8 ml to 15 ml, 1.0 ml to 10 ml, or 1.2 ml to 5 ml.

In one embodiment, the element comprises, preferably consists of glass or polymer, preferably polymer. In one embodiment, the element comprises or consists of cyclic olefin copolymer (COC) and/or cyclic olefin polymer (COP) and/or polypropylene (PP). In one embodiment, the element comprises or consists of cyclic olefin copolymer (COC).

**Silicone coating**

In one aspect, a method for coating an element is provided, preferably to obtain a coated element according to this disclosure, comprising the steps:

- providing an element comprising a surface;

- applying one or more layers, preferably the smoothing layer and the barrier layer, by performing a first coating process on at least part of the surface of the element to obtain a pre-coated element,

- performing a second coating process comprising the steps:

  x) providing a silicone-forming composition, wherein the silicone-forming composition comprises:

    i) a silicone compound suitable to form a silicone network after reaction;

    ii) a catalyst suitable to catalyze a network reaction of the silicone compound;

    iii) an inert silicone oil, wherein the inert silicone oil does not take part in the network reaction; and

    iv) a diluent,

  wherein the diluent comprises a silicon-containing compound, wherein the silicone-forming composition comprises 45 to 95 wt.% diluent, and

  y) applying the silicone-forming composition to the pre-coated element, and reacting the silicone-forming composition

- obtaining a coated element.

In one embodiment of the method, the second coating process, i.e. step y) is performed by curing the composition, preferably curing the composition by annealing to 50 °C to 300 °C, or 80 °C to 120 °C and/or by applying IR irradiation.

In one embodiment of the method, i.e. the second coating process, i.e. step x), the diluent comprises a silicon-containing organic compound with 6 silicon atoms or less.

In one embodiment of the method, i.e. the second coating process, i.e. step x), the diluent has a surface tension of 19 mN/m or less.

In one embodiment of the method, i.e. the second coating process, i.e. step x), the diluent is chosen from the list of:

- cyclic silicones, such as octamethylcyclotetrasiloxane, decamethylcyclopentasiloxane, dodecamethylcyclohexasiloxane, tetramethylcyclotetrasiloxane, pentamethylcyclopentasiloxane,

- hexamethyldisiloxane (HMDSO),

- octamethyltrisiloxane,

- decamethyltetrasiloxane.

In one embodiment of the method, i.e. the second coating process, i.e. step x), the silicone-forming composition has a viscosity between 0.5 to 200 m·Pas, 1 to 50 m·Pas, or 1 to 10 m·Pas, at a temperature of 23 °C. The viscosity can be measured using a rotational viscometer, e.g. described in DIN ISO 7884-2:1998-2.

In one embodiment of the method, i.e. the second coating process, i.e. step x), the silicone-forming composition has a contact angle of 28° or less, 24° or less, 18° or less, or 5° or less, 1 s after spreading the composition on a borosilicate glass surface at a temperature of 23 °C, wherein the contact angle has been determined by drop shape analysis.

**Coated container**

In one aspect according to this disclosure, a coated container is provided,

wherein the coated container comprises an inner surface and an outer surface,

wherein at least a part of the inner surface is coated by a coating; and

wherein the coated container satisfies the following equation:

$$i/o \leq d$$

a value d ($[mg/cm^2]/[mg/cm^2]$) of 0.90, or 0.80, or 0.70, or 0.60, or 0.50, or 0.40, or 0.30, or 0.20, or 0.14, or 0.10, or 0.07,

wherein i is the leaching ($mg/cm^2$) of one or more type(s) of ions and/or compounds obtainable by the method the method $L_i$ as described herein; and

wherein o is the leaching ($mg/cm^2$) of one or more type(s) of ions and/or compounds of the base material of the coated container and/or is obtainable by the method $L_o$ as described herein.

In one embodiment of the coated container, the one or more type(s) of ions and/or compounds are:

- one or more compound(s) comprising C, preferably one or more compound(s) comprising C except compounds comprising Si; and/or

- one or more monomer(s), preferably norbornene, norbornane, and/or bicyclopentane; and/or

- one or more antioxidant(s), preferably wherein the antioxidant(s) is/are selected from the group consisting of phenolic antioxidants, more preferably pentaerythritol tetrakis(3-(3,5-di-tert-butyl-4-hydroxyphenyl)propionate and/or dibutylhydroxytoluene (BHT); phosphite antioxidants, more preferably tris(2,4-ditert-butylphenyl)phosphite; phosphonite antioxidants, preferably tetrakis(2,4-di-tert-butylphenyl)[1,1-biphenyl]-4,4'-diylbisphosphonite; and thioether antioxidants, more preferably wherein the antioxidant(s) is/are phenolic antioxidant(s), more preferably pentaerythritol tetrakis(3-(3,5-di-tert-butyl-4-hydroxyphenyl)propionate.

In one embodiment, the coated container or the coated element comprise a base material, wherein the base material comprises

- one or more compound(s) comprising C, preferably one or more compound(s) comprising C except compounds comprising Si; and/or

- one or more monomer(s), preferably norbornene, norbornane, and/or bicyclopentane; and/or

- one or more antioxidant(s), preferably wherein the antioxidant(s) is/are selected from the group consisting of phenolic antioxidants, more preferably pentaerythritol tetrakis(3-(3,5-di-tert-butyl-4-hydroxyphenyl)propionate and/or dibutylhydroxytoluene (BHT); phosphite antioxidants, more preferably tris(2,4-ditert-butylphenyl)phosphite; phosphonite antioxidants, preferably tetrakis(2,4-di-tert-butylphenyl)[1,1-biphenyl]-4,4'-diylbisphosphonite; and thioether antioxidants, more preferably wherein the antioxidant(s) is/are phenolic antioxidant(s), more preferably pentaerythritol tetrakis(3-(3,5-di-tert-butyl-4-hydroxyphenyl)propionate.

**Method $L_i$**

In one embodiment of this disclosure, the following method (method $L_i$) is provided, comprising the steps:

- providing a container, preferably a coated container;

- in case the container exhibits two openings, e.g. a syringe: covering the smaller opening with a closure, e.g. a tip cap;

- filling 0.9 x (brimful volume) of a 0.005 mol/l KOH-solution (KOH: Potassium hydroxide hydrate ≥99.995%, Suprapur® (Merck), demineralized water (ultrapure water of purity 1 analogue DIN ISO 3696 with ≤ 0,1 μS/cm at 25°C)) in the pharmaceutical container;

- closing the container with a closure seal, e.g. aluminum foil or a stopper;

- inserting the container in an autoclave (e.g. Systec, model DX-150 (PM-CA-0001-01));

- treating the container for 3 hours at 121°C and 1 bar above ambient pressure, e.g. 2 bar;

- opening the closure seal;

- emptying the container;

- filling and rinsing the container two times with deionized water;

- in case the container is a glass container: filling 0.9 x (brimful volume) of 0.1 mol/l HCl-solution (hydrochloric acid 30% Suprapur® (Merck)) diluted with demineralized water (ultrapure water of purity 1 analogue DIN ISO 3696 with ≤ 0.1 μS/cm at 25°C)), i.e. the same volume as used in the alkaline treatment, in the pharmaceutical container, or

- in case the container is a polymer container: filling 0.9 x (brimful volume) of 0.1 mol/l HCl-solution (hydrochloric acid 30% Suprapur® (Merck)) diluted with demineralized water (ultrapure water of purity 1 analogue DIN ISO 3696 with ≤ 0.1 μS/cm at 25°C) and isopropanol (1:1 / vol%:vol%), i.e. the same volume as used in the alkaline treatment, in the pharmaceutical container;

- closing the container with a closure seal;

- inserting the container in an autoclave (e.g. Systec, model DX-150 (PM-CA-0001-01));

- treating the container for 6 hours at 121°C and 1 bar above ambient pressure;

- opening the closure seal;

- in case the container is a glass container: analyzing the content (mg/l) of one or more type(s) of ions and/or compounds, preferably [Na] ions, in the 0.1 mol/l HCl-solution by FAAS analysis, e.g. using Varian SpectrAA 280 FS (PE 3-004), to obtain the value [mg/l] for the leaching of one or more type(s) of ions and/or compounds, preferably [Na] ions;

- in case the container is a polymer container: analyzing the content (mg/l) of one or more type(s) of ions and/or compounds, preferably the antioxidant and/or organic compounds, more preferably the antioxidant, in the 0.1 mol/l HCl-solution by LC-MS, e.g. using Waters I-Class UPLC system with Waters Xevo qTOF, to obtain the value [mg/l] for the leaching of one or more type(s) of ions and/or compounds, preferably the antioxidant and/or organic compounds, more preferably the antioxidant;

- calculating the value (mg/cm$^2$) by the following formula:

$$A * B / C,$$

wherein A is the above value [mg/l] for the leaching of one or more type(s) of ions and/or compounds,
wherein B is 0.9 x brimful volume, and

wherein C is the wetted inner surface of the container (in cm$^2$); to obtain the value [mg/cm$^2$] for the leaching of one or more type(s) of ions and/or compounds, preferably Na ions or the antioxidant, i.e. the value i.

An alternative embodiment comprises a method (method $L_i$), comprising the steps:

- providing a container, preferably a coated container;

- in case the container exhibits two openings, e.g. a syringe: covering the smaller opening with a closure, e.g. a tip cap;

- filling 0.9 x (brimful volume) of a 0.005 mol/l KOH-solution (KOH: Potassium hydroxide hydrate ≥99.995%, Suprapur® (Merck), demineralized water (ultrapure water of purity 1 analogue DIN ISO 3696 with ≤ 0,1 μS/cm at 25°C)) in the pharmaceutical container;

- closing the container with a closure seal, e.g. aluminum foil or a stopper;

- inserting the container in an autoclave (e.g. Systec, model DX-150 (PM-CA-0001-01));

- treating the container for 3 hours at 121°C and 1 bar above ambient pressure, e.g. 2 bar;

- opening the closure seal;

- emptying the container;

- filling and rinsing the container two times with deionized water;

- filling 0.9 x (brimful volume) of 0.1 mol/l HCI-solution (hydrochloric acid 30% Suprapur® (Merck)) diluted with demineralized water (ultrapure water of purity 1 analogue DIN ISO 3696 with ≤ 0.1 μS/cm at 25°C)), i.e. the same volume as used in the alkaline treatment, in the pharmaceutical container;

- closing the container with a closure seal;

- inserting the container in an autoclave (e.g. Systec, model DX-150 (PM-CA-0001-01));

- treating the container for 6 hours at 121°C and 1 bar above ambient pressure;

- opening the closure seal;

- emptying the container;

- filling and rinsing the container two times with deionized water;

- drying the container, preferably with air;

- filling 0.9 x (brimful volume) of n-hexane, i.e. the same volume as used in the alkaline treatment, in the pharmaceutical container;

- shaking the pharmaceutical container in an orbital shaker (80 rpm) for 30 min at room temperature;

- analyzing the content (mg/l) of one or more type(s) of ions and/or compounds, preferably the antioxidant and/or organic compounds, more preferably the antioxidant, in the n-hexane-solution by LC-MS, e.g. using Waters I-Class UPLC system with Waters Xevo qTOF, to obtain the value [mg/l] for the leaching of one or more type(s) of ions and/or compounds, preferably the antioxidant and/or organic compounds, more preferably the antioxidant;

- calculating the value (mg/cm$^2$) by the following formula:

$$A * B / C,$$

wherein A is the above value [mg/l] for the leaching of one or more type(s) of ions and/or compounds,

wherein B is 0.9 x brimful volume, and

wherein C is the wetted inner surface of the container (in cm$^2$); to obtain the value [mg/cm$^2$] for

the leaching of one or more type(s) of ions and/or compounds, preferably Na ions or the antioxidant, i.e. the value i.

The value o may be obtained by several methods and preferably the value o is determined as described herein. The value o is either the content (mg/cm$^2$) of one or more type(s) of ions and/or compounds of the base material of the coated container and/or, preferably or, obtainable by the method $L_o$ as described herein, more preferably the value o is obtainable by the method $L_o$ as described herein.

If the same but uncoated container, which is used to obtain value i is available, then value o may be obtainable by method $L_i$ described above using the uncoated container to obtain the content (mg/cm$^2$) of one or more type(s) of ions and/or compounds of the base material of the coated container. If the values i and o should be obtained by only one container, value i is obtainable be the method $L_i$ above and value o is obtainable by the method $L_o$ below. Preferably, o is obtainable by the method $L_o$. The method $L_o$ can be used for a container, which is coated or uncoated on the outside, however, preferably the container is uncoated on the outside. Preferably, in the methods $L_i$ and $L_o$, the biggest opening faces upwards.

**Method $L_o$**

In one embodiment of this disclosure, the following method (method $L_o$) is provided, comprising the steps:

- providing a container, preferably the same container as used for the method $L_i$ above;

- in case the container exhibits two openings, e.g. a syringe: covering the smaller opening with a closure, e.g. a tip cap;

- filling 0.9 x (brimful volume) of a solution in the pharmaceutical container;

- closing the container with a closure seal;

- inserting the container in a beaker, e.g. a stainless steel or aluminum beaker;

- filling the beaker with a 0.005 mol/l KOH-solution (KOH: Potassium hydroxide hydrate ≥99.995%, Suprapur® (Merck), demineralized water (ultrapure water of purity 1 analogue DIN ISO 3696 with ≤ 0,1 μS/cm at 25°C)) till a level is reached which is the same as the level in the container;

- closing the beaker with a closure;

- inserting the beaker in an autoclave (e.g. Systec, model DX-150 (PM-CA-0001-01));

- treating the beaker for 3 hours at 121°C and 1 bar above ambient pressure, e.g. 2 bar;

- opening the closure of the beaker;

- emptying the beaker;

- filling and rinsing the beaker two times with deionized water and washing the outside of the container by rinsing the outer surface with deionized water;

- inserting the container again in the beaker, e.g. the stainless steel or aluminum beaker;

- in case the container is a glass container: filling the beaker with a 0.1 mol/l HCl-solution (hydrochloric acid 30% Suprapur® (Merck)) diluted with demineralized water (ultrapure water of purity 1 analogue DIN ISO 3696 with ≤ 0.1 μS/cm at 25°C)) )) till a level is reached which is the same as the level in the container, i.e. the same volume as used in the alkaline treatment, or

- in case the container is a polymer container: filling the beaker with a 0.1 mol/l HCl-solution (hydrochloric acid 30% Suprapur® (Merck)) diluted with demineralized water (ultrapure water of purity 1 analogue DIN ISO 3696 with ≤ 0.1 μS/cm at 25°C) and isopropanol (1:1 / vol%:vol%), till a level is reached which is the same as the level in the container, i.e. the same volume as used in the alkaline treatment;

- closing the beaker with a closure;

- inserting the beaker in an autoclave (e.g. Systec, model DX-150 (PM-CA-0001-01));

- treating the beaker for 6 hours at 121°C and 1 bar above ambient pressure;

- opening the beaker;

- in case the container is a glass container: analyzing the content (mg/l) of one or more type(s) of ions and/or compounds, preferably [Na] ions, in the 0.1 mol/l HCl-solution by FAAS analysis, e.g. using Varian SpectrAA 280 FS (PE 3-004), to obtain the value [mg/l] for the leaching of one or more type(s) of ions and/or compounds, preferably [Na] ions;

- in case the container is a polymer container: analyzing the content (mg/l) of one or more type(s) of ions and/or compounds, preferably the antioxidant and/or organic compounds, more preferably the antioxidant, in the 0.1 mol/l HCl-solution by LC-MS, e.g. using Waters I-Class UPLC system with Waters Xevo qTOF, to obtain the value [mg/l] for the leaching of one or more type(s) of ions and/or compounds, preferably the antioxidant and/or organic compounds, more preferably the antioxidant;

- calculating the value (mg/cm$^2$) by the following formula:

$$A * B / C,$$

wherein A is the above value [mg/l] for the leaching of one or more type(s) of ions and/or compounds,

wherein B is the volume of the solution in the beaker, and

wherein C is the wetted outer surface of the container (in cm$^2$); to obtain the value [mg/cm$^2$] for the leaching of one or more type(s) of ions and/or compounds, i.e. the value o.

Methods and Definitions

**ToF-SIMS (Time-of-Flight Secondary-Ion-Mass-Spectrometry)**

In the following the measuring method and the data evaluation of the specific TOF-SIMS measurement is explained in detail. The values for the ions, e.g. the ions [$C_3H_5^+$], [$C_4H_3^-$], [$SiC_2H_3^+$] and [$SiCH_3O^-$], can be obtained according to the following description.

Measuring method

For the measurement a TOF SIMS (TOF.SIMS 5 from Iontof) can be used. If not stated otherwise, the TOF-SIMS are measured according to ASTM E 1829 und ASTM E 2695.
The measurement has been started after establishing a vacuum of 1.0 mbar or less.
The following parameter settings were used for the TOF-SIMS:

For the analysis:

| | |
|---|---|
| primary ion: | Bi$^{3+}$; |
| energy: | 30 keV; |
| measuring area: | 100 $\times$ 100 $\mu$m$^2$; |
| pattern: | 128 $\times$ 128 random; and |
| bismuth analysis current: | 0.3 pA. |

For the sputter gun (Argon cluster source):

| | |
|---|---|
| sputter ion: | Ar-cluster; |
| energy: | 10 keV; |
| sputter area: | $300 \times 300 \ \mu m^2$; and |
| sputter current of Ar-clustersource: | 8.5 nA. |

Further experimental details:

| | |
|---|---|
| cycle time: | 200 $\mu s$ |
| analyzer extractor: | 2462 V (positive ions) and 2480 V (negative ions); |
| analyzer detector: | 9000 V; |
| charge compensation: | flood gun; |
| primary ion flight time correction: | on; and |
| gas flooding: | $9 \times 10^{-7}$ mbar |

A sample of a coated polymer container, for example one half of an on the inside coated container, which was cut lengthwise in two pieces, is positioned in such a way that the centerline of the sputter gun and the centerline of the liquid metal ion gun of TOF-SIMS hit the coated area of the sample so that the sputter area covers the entire measuring area, preferably that the centerline of the sputter gun and the centerline of the liquid metal ion gun of TOF-SIMS hit the same point of the coated area of the sample. The TOF-SIMS measures either positive ions or negative ions. To obtain both kinds of ions, two measurements can be conducted using for each measurement a new area of the same sample or a new sample, e.g. the first and the second half of a coated container, which is cut lengthwise in two pieces.

Determination of the layers

The layer structure of the coated element includes, in this order, a smoothing layer (SL), a barrier layer (BL), a protection layer (PL), and an optional gliding layer (GL), wherein the coated element has a polymer substrate. Each of the said layers is chemically distinct and is characterised by the detection of specific ToF-SIMS species, i.e. $SiC^-$, $C_4^-$, $C_4H_3^-$, $SiCH_3O^-$, and $SiO_3^-$ in negative mode, and $C_3H_5^+$, $SiO^+$. $SiC_2H^+$, $SiC_2H_3^+$, $Si_2O^+$, $SiC_3H_9^+$, and $CH_3^+$ in positive mode. The exact qualitative and quantitative characteristics depend on both the PICVD parameters employed during each of the four deposition steps (1 to 4) in addition to the chosen precursor, and the sputter and analysis parameters of the ToF-SIMS measurement. Figure 1B and Figure 1C, respectively, show the characteristic trend of the point-to-point normalisation intensity of selected ToF-SIMS species in positive and negative mode.

Boarder of layers

The order of layers built on top of the polymer substrate, in this order, are a smoothing layer (SL), a barrier layer (BL), a protection layer (PL), and an optional gliding layer (GL). Based on Example 1, and using the above sputter and analysis ToF-SIMS settings, the boarder(s) between the smoothing layer (SL), the barrier layer (BL), the protection layer (PL), and the optional gliding layer (GL), may be found or established on one or more of the following criteria:

$$[SiCH_3O^-]_{GL}/[SiO_3^-]_{GL} > 1.0;$$

$$[SiCH_3O^-]_{BL}/[SiO_3^-]_{BL} < 1.0;$$

$$[SiC_3H_9^+]_{GL}/[SiO^+]_{GL} > 1.1;$$

$$[SiC_3H_9^+]_{PL}/[SiO^+]_{PL} < 1.0;$$

$$[SiO^+]_{BL}/[SiC_2H^+]_{BL} > 1.1;$$

$[SiO^+]_{GL}/[SiC_2H^+]_{GL} < 1.1;$

$[SiO^+]_{PL}/[SiC_2H^+]_{PL} < 1.1;$ and

$[SiO^+]_{SL}/[SiC_2H^+]_{SL} < 1.1.$

Once the polymer substrate has been reached, i.e. after sputtering away all of the smoothing layer (SL), the barrier layer (BL), the protection layer (PL), and the optional gliding layer (GL), $C_3H_5^+$ and $C_4H_3^-$ as well as $C_4^-$ become the dominant ToF-SIMS species in positive and negative mode, whose point-to-point normalisation (intensities) are $>>$ 1.0 as compared to all Si-containing ToF-SIMS species.

**Thickness of the coating and its layers**

The thickness of the coating, as well as the individual layers, i.e. the smoothing layer, the barrier layer, the protection layer, and the gliding layer, is estimated by TEM (transmission electron microscopy). A Scanning Transmission Electron Microscope (SETM; Hitachi HF5000) is used at an accelerating voltage of 200 kV, at a capture resolution of 1024 x 1024 and an acquisition time of 20 seconds. Further instrument settings are: column mode operation (HR), DF-mode (ZCM), BF-mode (WAM), and STEM condenser aperture #2.

Sample preparation is conducted by first sputtering metallic titanium layer of 50 nm, to establish conductivity and surface protection, using a cold magnetron. Subsequent steps are performed using an Auriga 40 SEM/FIB cross beam: SEM-induced deposition of carbon was done using an organic precursor (for the protection from Ga ions), FIB-induced deposition of Pt using an organic precursor (for the protection of the surface during lamella preparation), at 30 kV Ga FIB to cut the lamella, sample transfer to a copper grid, thinning with 30 kV Ga ions, and final thinning with 2 kV Ga ions. This typically results in a final lamella width of 20 $\mu$m, and a final thickness of the titanium layer ca. 50 nm.

**Roughness of the uncoated surface of the element**

The surface roughness depth SRz has been determined based on and according to ISO 25178 - 2:2012 using a white light interferometer (WLI) on an uncoated inner surface of the element as well as an uncoated outer surface of the element. A WLI from Zygo (type NexView) was used, and a field-of-view of 400 $\mu$m $\times$ 400 $\mu$m was measured and corrected for the curvature of the element before any of the subsequent calculations. To obtain SRz parameters, a surface area was analyzed by fitting a minimum enclosing rectangle and applying a 5 x 5 sampling grid, for a total of 25 sampling areas. All sampling areas together make up the evaluation area. Whereas Rz refers to the roughness obtained from an individual line scan (across 5 points), SRz reflects the average radial peak-to-valley areal roughness on a surface, i.e. the average of the largest half of many individual Rz results is determined by slicing the areal data array about its center through 360 degrees. The Rz results are sorted by magnitude and SRz is calculated by averaging the largest 50% of the Rz values. A line-generation algorithm is used to determine the actual pixel-to-pixel path of each slice, and there is no interpolation between pixels. SRz thus covers the entire array, and due to its radial generation it is lay independent.

Depending on the type of container, several measurements were performed and averaged over a well-defined number of containers and a well-defined number of measured positions.

For a syringe (COC, 1 ml long), 10 containers were measured at 6 different positions, giving a SRz of 132 nm $\pm$ 109 nm (mean $\pm$ standard deviation). The 6 different positions were measured at a distance from the stamp of 10 mm, 25 mm, and 40 mm, and at opposite positions of 180° with respect to the central axis of the syringe.

For a vial (COP, 2 ml), 10 containers were measured at 3 different positions, giving a SRz of 147 nm $\pm$ 118 nm (mean $\pm$ standard deviation). The 3 different positions were measured at a vial height of 10 mm and at angular positions of 120° with respect to the central axis of the vial.

**Gliding force**

Coefficients of static and gliding friction for a gliding layer were determined by pressing a V9361 FM457/0 FLNC2 057 stopper from Datwyler Pharma Packaging with an external diameter of 6.9 $\pm$ 0.1 mm into a corresponding syringe at a speed of 100 mm/min. The forces required for this pressing action were recorded.

The coefficients of static and sliding friction are ascertained by moving a suitable stopper, immediately after insertion into the hollow body, at a constant speed of 100 mm/min through the hollow body and measuring the force required for this as a function of the insertion depth. Typical static/sliding friction diagrams have a linear rise in force at the start of movement of the stopper. As soon as the force of static friction has been overcome, the stopper begins to move and there is sliding of the stopper through the cylinder which, in the case of a good sliding layer, requires a relatively constant pushing force. Immediately prior to onset of the sliding movement of the stopper there is generally a maximum in the static/sliding friction diagram, which represents the coefficient of static friction. The relatively constant force during the sliding movement represents the coefficient of sliding friction. The static/sliding friction diagrams should typically be measured with a stopper that is also used as the stopper for the primary pharmaceuticals packaging and with which a corresponding leak tightness is achieved, without however requiring too great an effort.

**Breaking force**

The breaking force shall be understood as the static friction (force) measured at the surface of a coated element or an uncoated element.

The breaking force was measured in 1 ml long COC syringes with a siliconized stopper from Datwyler Pharma 257/2 ISAF2. The breaking force was measured as the maximal force along the initial path of 4 mm. After that initial path was passed, monitoring over the further path of 30 mm gave access to an average gliding force. Measurement was performed under dry conditions, after storing the stopper for 24 hours in the syringe at a temperature of 20 °C. Further test parameters were: 100 N load cell, 100 mm/min test speed, plunger rod without thread.

**Examples**

**Example 1 (inventive example)**

A resin syringe (1 ml long COC) was provided and was coated subsequently in four steps without a vacuum break using an apparatus according to WO 2007/022976 A2 (Fig. 1). The plasma was driven by a microwave irradiation having a frequency of 2.45 GHz. The plasma reaction chamber was mainly the inside of the syringe and ambient conditions prevailed outside of the syringe. Before starting the film depositions the reaction chamber was evacuated until a value of 0.05 mbar was reached.

Four separate, subsequent coating steps were applied:
In a first step a gasflow of 20 sccm HMDSO was adjusted at a pressure of 0.66 mbar. Afterwards a plasma was excited with an input power of 1460 W in a pulsed mode with a pulse duration of 0.5 ms and pulse pause of 10 ms. The plasma treatment was performed for 5 seconds.

In a second step a gasmixture of 0.6 sccm HMDSO and 39.4 sccm Oxygen was adjusted at a pressure of 0.36 mbar. Afterwards a plasma was excited with an input power of 1180 W in a pulsed mode with a pulse duration of 2 ms and pulse pause of 5 ms. The plasma treatment was performed for 9 seconds.

In a third step a gasmixture of 14 sccm HMDSO and 6 sccm Oxygen was adjusted at a pressure of 0.66 mbar. Afterwards a plasma was excited with an input power of 1420 W in a pulsed mode with a pulse duration of 0.5 ms and pulse pause of 10 ms. The plasma treatment was performed for 1 seconds.

In a fourth step a gasflow of 10 sccm HMDSO was adjusted at a pressure of 0.84 mbar. Afterwards a plasma was excited with an input power of 980 W in a pulsed mode with a pulse duration of 0.04 ms and pulse pause of 1 ms. The plasma treatment was performed for 9 seconds. At the end the reaction chamber was vented with nitrogen until atmospheric pressure was reached.

**Example 2 (inventive example)**

A resin syringe (1 ml long COC) was provided and was coated according to Example 1, except that the fourth coating step was omitted. A further coating was performed by employing a liquid coating according to Example 2 of EP 3636303 A1. The liquid coating was prepared by charging 80 g of a vinyl-functionalized polydimethylsiloxane in a reaction vessel and admixing with 640 g of hexamethyldisiloxane. Under constant stirring at 1000 rpm, 2 g of methylhydrosiloxane/dimethylsiloxane copolymer, 48 g of liquid polydimethylsiloxane, 1.1 g of 1,1,3,3-tetramethyl-1,3-divinyldisiloxane-complexed platinum and 0.1 g of butynol as inhibitor were added to this reaction mixture. The preparation can be used after a stirring time of 60 s.

The preparation was applied to the plastics hollow body on the inner side by a wiping process and cured by heating to 250 °C for 3.5 seconds. A 1-ml COC syringe of the "1-ml long" standard size with an internal diameter of 6.5 mm was used as substrate for depositing the gliding layer.

Coefficients of static and gliding friction for the cured gliding layer were subsequently determined. This involved pressing the V9361 FM457/0 FLNC2 057 stopper from Datwyler Pharma Packaging having an external diameter of 6.9 ± 0.1 mm into the syringe at a speed of 100 mm/min. The forces required for this were recorded. The coefficients for both static and gliding friction were less than 10 N.

**Reference Example**

A resin syringe (1 ml long COC) was provided and was coated according to Example 1, except that the fourth coating step was omitted.

**Claims**

1.  Coated element having a surface, wherein at least a part of the surface of the element is coated by a coating, wherein the coating comprises, in this order:

    - a smoothing layer (SL);
    - a barrier layer (BL); and
    - a protection layer (PL),

    wherein the smoothing layer satisfies the condition $a1_{SL} \leq [Si_2O^+]_{SL}/[SiC_3H_9^+]_{SL} \leq a2_{SL}$, wherein $a1_{SL}$ is 1.0, wherein $a2_{SL}$ is $1*10^5$, wherein $[SiC_3H_9^+]_{SL}$ are the counts of $[SiC_3H_9^+]$ ions in the smoothing layer, measured by a TOF-SIMS, wherein $[Si_2O^+]_{SL}$ are the counts of $[Si_2O^+]$ ions in the smoothing layer, measured by a TOF-SIMS, and/or

    wherein the barrier layer satisfies the condition $a1_{BL} \leq [Si_2O^+]_{BL}/[SiC_3H_9^+]_{BL} \leq a2_{BL}$, wherein $a1_{BL}$ is 1, wherein $a2_{BL}$ is $1*10^5$, wherein $[SiC_3H_9^+]_{BL}$ are the counts of $[SiC_3H_9^+]$ ions in the barrier layer, measured by a TOF-SIMS, and wherein $[Si_2O^+]_{BL}$ are the counts of $[Si_2O^+]$ ions in the barrier layer, measured by a TOF-SIMS, and/or wherein the protection layer satisfies the condition $a1_{PL} \leq [Si_2O^+]_{PL}/[SiC_3H_9^+]_{PL} \leq a2_{PL}$, wherein $a1_{PL}$ is 1.0, wherein $a2_{PL}$ is $1*10^{10}$, wherein $[SiC_3H_9^+]_{PL}$ are the counts of $[SiC_3H_9^+]$ ions in the protection layer, measured by a TOF-SIMS; and wherein $[Si_2O^+]_{PL}$ are the counts of $[Si_2O^+]$ ions in the protection layer, measured by a TOF-SIMS,

    wherein the coated element optionally has a gliding force of 0.1 to 10.0 N, and/or a breaking force of 1 N to 18 N.

2.  Coated element having a surface, wherein at least a part of the surface of the element is coated by a coating, wherein the coating comprises, in this order:

    - a smoothing layer (SL);
    - a barrier layer (BL); and
    - a protection layer (PL),

    wherein the smoothing layer has a thickness of 1 nm or more, and/or

    wherein the barrier layer has a thickness of 1 nm or more, and/or
    wherein the protection layer has a gliding force of 0.1 to 10.0 N, and/or a breaking force of 1 N to 18 N, and/or
    wherein the coated element has a gliding force of 0.1 to 10.0 N, and/or a breaking force of 1 N to 18 N.

3.  Coated element according to claim 1 or claim 2, further comprising a gliding layer (GL),

    wherein the gliding layer is adjacent to the protection layer,
    wherein the gliding layer satisfies the condition $a1_{GL} \leq [SiC_3H_9^+]_{GL}/[Si_2O^+]_{GL} \leq a2_{GL}$, wherein $a1_{GL}$ is 1.1, wherein $a2_{GL}$ is $1*10^{10}$, wherein $[SiC_3H_9^+]_{GL}$ are the counts of $[SiC_3H_9^+]$ ions in the gliding layer, measured by a TOF-SIMS; and wherein $[Si_2O^+]_{GL}$ are the counts of $[Si_2O^+]$ ions in the gliding layer, measured by a TOF-SIMS.

4.  Coated element according to any one of claims 1 to 3, wherein the element comprises, preferably consists of glass or polymer, preferably polymer, more preferably cyclic olefin copolymer (COC) and/or cyclic olefin polymer (COP) and/or polypropylene (PP), more preferably cyclic olefin copolymer (COC).

5.  Coated element according to claim 3 or claim 4, wherein the gliding layer satisfies one or more of the following parameters:

    - $a1_{GL} \leq [SiC_3H_9^+]_{GL}/[Si_2O^+]_{GL} \leq a2_{GL}$, wherein $a1_{GL}$ is 1.1, wherein $a2_{GL}$ is $1*10^{10}$, wherein $[SiC_3H_9^+]_{GL}$ are the counts of $[SiC_3H_9^+]$ ions in the gliding layer, measured by a TOF-SIMS; and wherein $[Si_2O^+]_{GL}$ are the counts of $[Si_2O^+]$ ions in the gliding layer, measured by a TOF-SIMS, and

- $b1_{GL} \leq [SiC_2H^+]_{GL}/[SiO^+]_{GL} \leq b2_{GL}$, wherein $b1_{GL}$ is 1.1, wherein $b2_{GL}$ is 1000, wherein $[SiC_2H^+]_{GL}$ are the counts of $[SiC_2H^+]$ ions in the gliding layer, measured by a TOF-SIMS; and wherein $[SiO^+]_{GL}$ are the counts of $[SiO^+]$ ions in the gliding layer, measured by a TOF-SIMS.

6. Coated element according to any one of the preceding claims, wherein the protection layer satisfies one or more of the following parameters:

- $a1_{PL} \leq [Si_2O^+]_{PL}/[SiC_3H_9^+]_{PL} \leq a2_{PL}$, wherein $a1_{PL}$ is 1.0, wherein $a2_{PL}$ is $1*10^{10}$, wherein $[SiC_3H_9^+]_{PL}$ are the counts of $[SiC_3H_9^+]$ ions in the protection layer, measured by a TOF-SIMS; and wherein $[Si_2O^+]_{PL}$ are the counts of $[Si_2O^+]$ ions in the protection layer, measured by a TOF-SIMS, and
- $b1_{PL} \leq [SiC_2H^+]_{PL}/[SiO^+]_{PL} \leq b2_{PL}$, wherein $b1_{PL}$ is 0.5, wherein $b2_{PL}$ is $1*10^5$, wherein $[SiC_2H^+]_{PL}$ are the counts of $[SiC_2H^+]$ ions in the protection layer, measured by a TOF-SIMS; and wherein $[SiO^+]_{PL}$ are the counts of $[SiO^+]$ ions in the protection layer, measured by a TOF-SIMS.

7. Coated element according to any one of the preceding claims, wherein the barrier layer satisfies one or more of the following parameters:

- $a1_{BL} \leq [Si_2O^+]_{BL}/[SiC_3H_9^+]_{BL} \leq a2_{BL}$, wherein $a1_{BL}$ is 1, wherein $a2_{BL}$ is $1*10^5$, wherein $[SiC_3H_9^+]_{SL}$ are the counts of $[SiC_3H_9^+]$ ions in the barrier layer, measured by a TOF-SIMS, and wherein $[Si_2O^+]_{BL}$ are the counts of $[Si_2O^+]$ ions in the barrier layer, measured by a TOF-SIMS, and
- $b1_{BL} \leq [SiO^+]_{BL}/[SiC_2H^+]_{BL} \leq b2_{BL}$, wherein $b1_{BL}$ is 1, wherein $b2_{BL}$ is $1*10^5$, wherein $[SiC_2H^+]_{BL}$ are the counts of $[SiC_2H^+]$ ions in the barrier layer, measured by a TOF-SIMS, wherein $[SiO^+]_{BL}$ are the counts of $[SiO^+]$ ions in the barrier layer, measured by a TOF-SIMS.

8. Coated element according to any one of the preceding claims, wherein the smoothing layer satisfies one or more of the following parameters:

- $a1_{SL} \leq [Si_2O^+]_{SL}/[SiC_3H_9^+]_{SL} \leq a2_{SL}$, wherein $a1_{SL}$ is 1.0, wherein $a2_{SL}$ is $1*10^5$, wherein $[SiC_3H_9^+]_{SL}$ are the counts of $[SiC_3H_9^+]$ ions in the smoothing layer, measured by a TOF-SIMS, wherein $[Si_2O^+]_{SL}$ are the counts of $[Si_2O^+]$ ions in the smoothing layer, measured by a TOF-SIMS, and
- $b1_{SL} \leq [SiC_2H^+]_{SL}/[SiO^+]_{SL} \leq b2_{SL}$, wherein $b1_{SL}$ is 1.0, wherein $b2_{SL}$ is $1*10^5$, wherein $[SiC_2H^+]_{SL}$ are the counts of $[SiC_2H^+]$ ions in the smoothing layer, measured by a TOF-SIMS, wherein $[SiO^+]_{SL}$ are the counts of $[SiO^+]$ ions in the smoothing layer, measured by a TOF-SIMS.

9. Coated element according to any one of the preceding claims, wherein the thickness of the smoothing layer is 1 nm to 20000 nm, or 5 nm to 5000, or 100 nm to 2000nm, or 150 nm to 1500nm, or 200 nm to 1000nm, or 250 nm to 500nm.

10. Coated element according to any one of the preceding claims,

wherein the time a sputter gun needs to reach the surface of the element is 0.5 minutes to 150 minutes, or 30 to 120 minutes, or 50 to 100 minutes; and/or
wherein in the case positive ions are measured, the point, when the sputter gun reaches the surface of the element is the point, when the counts of $[Si_2O^+]$ ions are equal to the counts of $[C_3H_5^+]$ ions; and/or
wherein in the case negative ions are measured, the point, when the sputter gun reaches the surface of the element is the point, when the counts of $[C_4H_3^-]$ ions are equal to the counts of $[SiO_3^-]$ ions; and/or
wherein the counts of the ions, preferably the positive and negative ions are obtainable by the method described in the description.

11. Coated element according to any one of the preceding claims,

wherein the element is a pharmaceutical container comprising an inner surface, wherein the inner surface is coated,
wherein the smoothing layer is in direct contact with the coated element,
wherein the gliding layer, if present, is the outermost layer.

12. Coated element according to any one of the preceding claims,

wherein one or more of the following conditions is/are fulfilled:

$[SiO_3^-]_{GL} < [SiO_3^-]_{BL};$

$[SiO_3^-]_{PL} < [SiO_3^-]_{BL};$

$[SiO_3^-]_{SL} < [SiO_3^-]_{BL};$ and/or

$[SiO_3^-]_{GL} < [SiO_3^-]_{PL},$

wherein $[SiO_3^-]_{GL}$ are the counts of $[SiO_3^-]$ ions in the gliding layer, measured by a TOF-SIMS;
wherein $[SiO_3^-]_{PL}$ are the counts of $[SiO_3^-]$ ions in the protection layer, measured by a TOF-SIMS;
wherein $[SiO_3^-]_{BL}$ are the counts of $[SiO_3^-]$ ions in the barrier layer, measured by a TOF-SIMS;
wherein $[SiO_3^-]_{SL}$ are the counts of $[SiO_3^-]$ ions in the smoothing layer, measured by a TOF-SIMS.

13. System or kit, comprising:

the coated element according to any one of the preceding claims, wherein the element is a syringe or cartridge;
a plunger, preferably a plunger rod; and
optionally a closure, preferably a tip cap and/or a needle shield.

14. Closed system comprising:

the coated element according to any one of claims 1 to 12, wherein the element is a syringe or cartridge;
a plunger, preferably a plunger rod; and
a closure, preferably a tip cap and/or a needle shield,

wherein the closed coated element passes the container closure integrity test according to DIN EN ISO 8871-5:2016; chapter 4.4 in combination with Annex D.

15. Closed system according to claim 14, comprising a composition, preferably a pharmaceutical composition, preferably a composition containing biologics or mRNA.

16. Method for coating an element, preferably a coated element according to any one of claims 1 to 12, comprising the steps:

(1) providing an element comprising a surface;
(2) applying a smoothing layer;
(3) applying a barrier layer;
(4) applying a protection layer; and
(5) optionally applying a gliding layer,

wherein each of the steps (2) to (4) comprises surrounding at least part of the surface of the element with a precursor, and

wherein each of the steps (2) to (4) comprises irradiating the precursor to generate a plasma using a PICVD method,
wherein the step (3) comprises establishing a flow rate of $O_2$ of 10 sccm or more during the irradiation of the precursor.

| | GL |
| --- | --- |
| | PL |
| | BL |
| | SL |
| CE | |

**Figure 1A**

Figure 1B

Figure 1C

Figure 2A

Figure 2B

Figure 2C

**Figure 2D**

Figure 2E

Figure 2F

Figure 2G

Figure 3A

**Figure 3B**

**Figure 3C**

**Figure 3D**

Figure 3E

Europäisches
Patentamt

European
Patent Office

Office européen
des brevets

# EUROPEAN SEARCH REPORT

Application Number

EP 22 18 7836

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/036147 A2 (SIO2 MEDICAL PRODUCTS INC [US]) 17 February 2022 (2022-02-17) * figures 1-5 * * paragraphs [0022], [0050], [0051], [0120], [0121], [0287] * ----- | 1-16 | INV. C23C16/40 C23C16/515 C23C14/04 |
| X | US 2015/297800 A1 (WEIKART CHRISTOPHER [US] ET AL) 22 October 2015 (2015-10-22) * figures 8-10 * * paragraphs [0111], [0401], [0515], [0727] - [0738]; example 10 * ----- | 1-16 | |

TECHNICAL FIELDS
SEARCHED (IPC)

C23C

The present search report has been drawn up for all claims

| Place of search | Date of completion of the search | Examiner |
|---|---|---|
| The Hague | 10 January 2023 | Schuhmacher, Jörg |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...............
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 18 7836

This annex lists the patent family members relating to the patent documents cited in the above-mentioned European search report.
The members are as contained in the European Patent Office EDP file on
The European Patent Office is in no way liable for these particulars which are merely given for the purpose of information.

10-01-2023

| Patent document cited in search report | | Publication date | Patent family member(s) | | | Publication date |
|---|---|---|---|---|---|---|
| WO 2022036147 | A2 | 17-02-2022 | NONE | | | |
| US 2015297800 | A1 | 22-10-2015 | US | 2015297800 | A1 | 22-10-2015 |
| | | | US | 2021330869 | A1 | 28-10-2021 |

EPO FORM P0459

For more details about this annex : see Official Journal of the European Patent Office, No. 12/82

**REFERENCES CITED IN THE DESCRIPTION**

**Patent documents cited in the description**

- WO 2007022976 A2

- EP 3636303 A1